# EUROPEAN PATENT APPLICATION

(11) **EP 1 715 052 A1**
(43) Date of publication of application: **25.10.2006**
(21) Application number: 05709422.9
(22) Date of filing: 27.01.2005
(51) Int. Cl.: C12N 15/87, A61K 48/00, C07K 16/28

(54) **COMPOSITION AND METHOD FOR ELEVATING GENE TRANSFER EFFICIENCY**

(30) Priority: 29.01.2004 JP 2004022315
(71) Applicant: National Institute of Advanced Industrial Science and Technology, Tokyo 100-8921 (JP); Cytopathfinder, Inc., Tokyo 142-0084 (JP)
(72) Inventor: MIYAKE, Masato, Amagasaki-shi, Hyogo 6610974 (JP); YOSHIKAWA, Tomohiro, Amagasaki-shi, Hyogo 6610974 (JP); UCHIMURA, Eiichiro, Amagasaki-shi, Hyogo 6610974 (JP); MIYAKE, Jun, Amagasaki-shi, Hyogo 6610974 (JP)
(74) Representative: Bates, Philip Ian
(86) International application number: PCT/JP2005/001148
(87) International publication number: WO 2005/073385

(57) **Abstract**

It is intended to provide a method whereby transfer efficiency can be improved under any conditions in the case of transferring (in particular, transfecting) a target substance that cannot easily be transferred into cells (for example, a DNA, a polypeptide, a sugar, a complex thereof or the like). Namely, a composition for elevating the transfer efficiency of a target substance into cells which contains a cell adhesion-related factor, and a device and a method using the composition.

## Description

### TECHNICAL FIELD

The present invention relates to the field of cell biology. More particularly, the present invention relates to a compound, composition, device, method and system for increasing the efficiency of introducing a substance into a cell.

### BACKGROUND ART

Techniques for introducing a protein into cells (i.e., transfection, transformation, transduction, etc.) are generally used in a wide variety of fields, such as cell biology, genetic engineering, molecular biology, and the like.

Transfection is conducted to temporarily express a gene in cells, such as animal cells and the like, so as to observe an influence of the gene. Since the advent of the post-genomic era, transfection techniques are frequently used to elucidate the functions of genes encoded by the genome.

Various techniques and agents used therein have been developed to achieve transfection. One technique employs a cationic substance, such as a cationic polymer, a cationic lipid, or the like, and is widely used.

In many cases, however, use of conventional agents is not sufficient for transfection efficiency. There has been conventionally no agent, which can be used either in solid phase or in liquid phase. Therefore, there is great demand for such an agent. Further, there is an increasing demand for a technique for efficiently introducing (e.g., transfecting, etc.) a target substance into cells or the like on a solid phase, such as microtiter plates, arrays, and the like.

The difficulty in transfecting cells or producing transgenic organisms hinders the progression of development of dominant negative screening in mammals. To overcome this problem, high-efficiency retrovirus transfection has been developed. Although this retrovirus transfection is potent, it is necessary to produce DNA to be packaged into viral intermediates, and therefore, the applicability of this technique is limited. Alternatively, high-density transfection arrays are being developed, but are not necessarily applicable to all cells. Various systems for liquid phase transfection have been developed. However, the efficiency of such techniques is low for adherent cells, for example. Thus, such techniques are not necessarily applicable to all cells.

As such, that the development of a transfection system which is applicable to any system or cell is greatly desired in the art. Demand for such a transfection system for use in mass high-through put assay using e.g., microtiterplates, arrays and the like, increases year on year, since such a system could be applied to a variety of cells and experimental systems.

### DISCLOSURE OF INVENTION

### (Problems to be solved by the invention)

Considering the above, it is an object of the present invention to develop a method for improving the introduction (in particular, transfection) efficiency of a target substance (e. g. DNA, polypeptide, sugar or a complex thereof or the like), which is difficult to introduce into a cell by means of conventional diffusion or hydrophobic interaction, under all circumstances in which it is desirable to introduce such.

### (Means for solving the problems)

Such an object has been fulfilled by the unexpected discovery that a system using a cellular adhesion related agent, such as an antibody against an integrin, significantly enhances the introduction efficiency of a target substance into a cell. Such an object has also been in part achieved by the discovery ,that the inhibition of adhesion of a cell unexpectedly enhances the introduction of a target substance such as a gene, and also in the case of genetic materials and the like, that expression of such in the cell is observed.

Accordingly, the present invention provides the following:
1. A composition for enhancing the introduction efficiency of a target substance into a cell, comprising a cellular adhesion related agent.
2. A composition for enhancing the introduction efficiency of a target substance into a cell according to item 1, wherein the cellular adhesion related agent comprises an interaction substance interacting with a cellular adhesion molecule.
3. A composition according to item 2, wherein the cellular adhesion molecule is an extracellular matrix.
4. A composition according to item 2, wherein the cellular adhesion molecule is an integrin receptor.
5. A composition according to item 2, wherein the cellular adhesion molecule comprises an RGD molecule.
6. A composition according to item 2, wherein the interaction molecule raises an antigen-antibody reaction with a partner of the cellular adhesion molecule.
7. A composition according to item 2, wherein the interaction molecule is an antibody or a derivative thereof.
8. A composition according to item 2, wherein the interaction molecule is a monoclonal or polyclonal antibody.
9. A composition according to item 2, wherein the interaction molecule comprises an antibody selected from the group consisting of an anti-CD49a antibody, an anti-CD49b antibody, an anti-CD49c antibody, an anti-CD49e antibody, and an anti-CD49f antibody.
10. A composition according to item 1, wherein the target substance comprises a genetic material.
11. A composition according to item 1, wherein the target substance comprises a nucleic acid molecule.
12. A composition according to item 1, wherein the target substance comprises DNA.
13. A composition according to item 4, wherein the integrin receptor is selected from the group consisting of CD49a, CD49b, CD49c, CD49d, CD49e, CD49f and CD29.
14. A composition according to item 4, wherein the integrin receptor is selected from the group consisting of CD29, CD49a, CD49c, Cd49d, CD49e and CD49f.
15. A composition according to item 4, wherein the integrin receptor interacts with a molecule selected from the group consisting of collagen, fibronectin, vitronectin and laminin.
16. A composition according to item 1, wherein the cell comprises at least one cell selected from the group consisting of a stem cell and a differentiated cell.
17. A composition according to item 1, wherein the cellular adhesion molecule is specifically expressed in the cell.
18. A composition according to item 1, wherein the target substance is a genetic material and the composition further comprises a gene introduction reagent.
19. A composition according to item 18, wherein the gene introduction reagent is selected from the group consisting of a cationic macromolecule, cationic lipid and calcium phosphate.
20. A composition according to item 1, further comprising a particle.
21. A composition according to item 20, wherein the particle comprises a gold colloid.
22. A composition according to item 1 further comprising a salt.
23. A composition according to item 22, wherein the salt is selected from the group consisting of salts comprised in a buffer and salts comprised in media.
24. A kit for enhancing gene introduction efficiency, comprising:
   (a) a cellular adhesion related agent; and
   (b) a gene introduction reagent.
25. A composition for introducing a target material to a cell, comprising:
   (A) a target material; and
   (B) a cellular adhesion related agent.
26. A composition according to item 25, wherein the target material comprises a substance selected from the group consisting of DNA, RNA, polypeptide, sugar and a complex thereof.
27. A composition according to item 25, wherein the target material comprises a DNA encoding a gene sequence to be transfected into the cell.
28. A composition according to item 25 further comprising a gene introduction reagent.
29. A composition according to item 25, wherein the cellular adhesion related agent comprises an interaction substance interacting with a cellular adhesion molecule.
30. A composition according to item 25, wherein the cellular adhesion related agent comprises an antibody to a cellular adhesion molecule.
31. A composition according to item 25 which is present as a liquid phase.
32. A composition according to item 25 which is present as a solid phase.
33. A device for enhancing gene introduction efficiency of a target molecule into a cell, comprising:
   (a) a target molecule; and
   (b) a cellular adhesion related agent,
   wherein the cellular adhesion related agent is immobilized onto a support.
34. A device according to item 33, wherein the target substance comprises a substance selected from the group consisting of DNA, RNA, polypeptide, sugar and a complex thereof.
35. A device according to item 33, wherein the target substance comprises a DNA encoding a gene sequence for the transfection. In particular, collagen IV has been elucidated to have particularly significant effects upon transfection efficiency.

### (Example 3: Improvement in transfection efficiency in solid phase)

### (Protocol)

The final concentration of DNA was adjusted to 1 µg/µL. A cellular adhesion related agent was preserved as a stock having a concentration of 200 µg/mL in ddH₂O. All dilutions were made using PBS, ddH₂O, or Dulbecco's MEM. A series of dilutions, for example, 0.2 µg/mL, 0.067 µg/mL, 0.04 µg/mL, 1.0 µg/mL, and the like, were formulated.

Transfection reagents were used in accordance with instructions provided by each manufacturer.

Plasmid DNA was removed from a glycerol stock and amplified in 100 mL L-amp overnight. Qiaprep Miniprep or Qiagen Plasmid Purification Maxi was used to purify DNA in accordance with a standard protocol provided by the manufacturer.

In the present Example, the following cells were used to confirm an effect: HepG2 cells (RCB1648, RIKEN Cell Bank, JPN). These cells were cultured in DMEM/10% IFS containing L-glut and pen/strep.

### (Dilution and DNA spotting)

Transfection reagents and DNA were mixed to form a DNA-transfection reagent complex. The complex formation requires a certain period of time. Therefore, the mixture was spotted onto a solid phase support (e.g., a poly-L-lysine slide) using a pipette.

For complex formation and spot fixation, the slides were dried overnight in a vacuum dryer.

Cellular adhesion related agents may be used when forming the above-described complexes, but in the present Example, they were physically coated onto a solid support such as a poly-L-lysine slide. Solutions reconstituted with such a antibody-cellular adhesion related agents at a designated use and volume were diluted to 100 to 5,000 fold with PBS, and thereafter placed onto the surface of glass slides and dried. The plates were extensively washed with PBS, and a complex of a gene introduction and a gene was printed thereupon. The cells of interest were plated onto these glass slides to conducting solid phase gene introduction.

### (Formulation of mixed solution and application to solid phase supports)

### (Distribution of cells)

Next, a protocol for adding cells will be described. Cells were distributed for transfection. The distribution was typically performed by low-pressure aspiration in a hood. A slide was placed on a dish, and a solution containing cells was added to the dish for transfection. The cells were distributed as follows.

The growing cells were adjusted to a concentration of 10⁷ cells/25 mL. The cells were plated on the slide in a 100x100x15 mm square Petri dish or a 100 mm (radius) × 15 mm circular dish. Transfection was conducted for about 40 hours. This period of time corresponded to about 2 cell cycles. The slide was treated for immunofluorescence.

### (Evaluation of gene introduction)

Gene introduction was evaluated by detection using, for example, immunofluorescence, fluorescence microscope examination, laser scanning, radioactive labels, and sensitive films, or emulsion.

When an expressed protein to be visualized is a fluorescent protein, such a protein can be observed with a fluorescence microscope and a photograph thereof can be taken. For large-sized expression arrays, slides may be scanned using a laser scanner for storage of data. If an expressed protein can be detected using fluorescent antibodies, an immunofluorescence protocol can be performed. If detection is based on radioactivity, the slide may be prepared as described above, and autoradiography using film or emulsion can be performed to detect radioactivity.

### (Laser scanning and Quantification of fluorescence intensity)

To quantify transfection efficiency, the present inventors used a DNA microarray scanner (GeneTAC UC4x4, Genomic Solutions Inc., MI). Total fluorescence intensity (arbitrary unit) was measured, and thereafter, fluorescence intensity per unit surface area was calculated.

### (RESULTS)

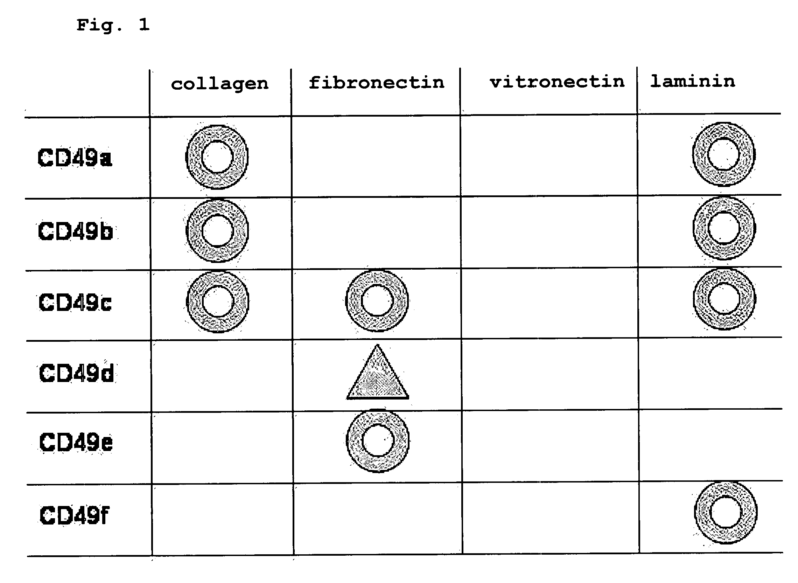

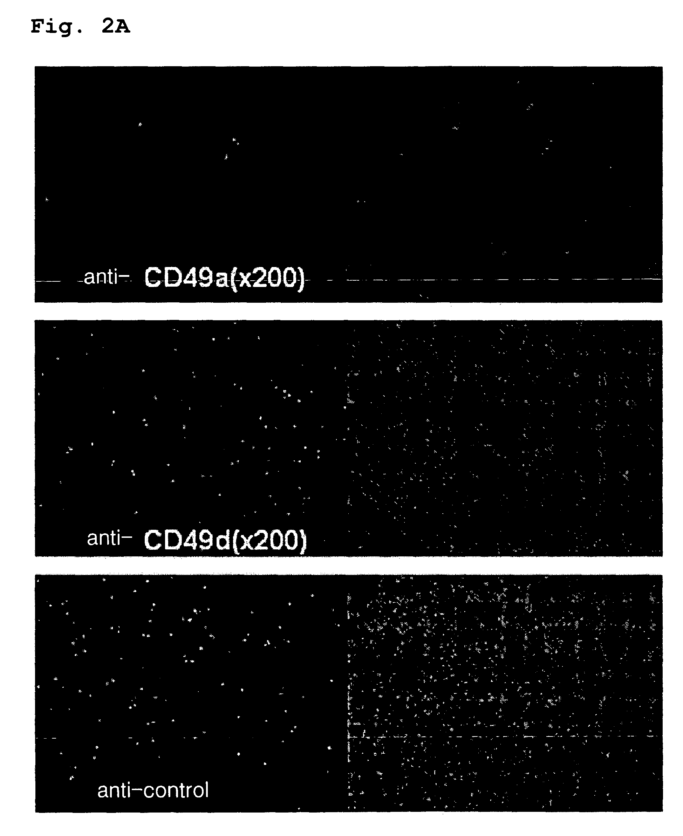

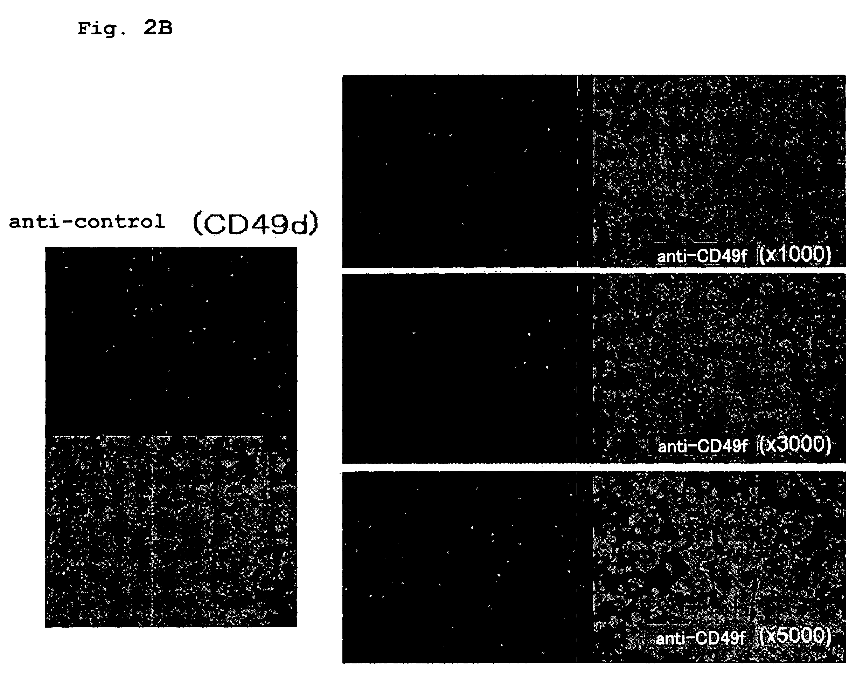

Figure **2A** depicts the results of experiments using a variety of cellular adhesion related agents and fibronectin when using HepG2 cells, as an example. Figure **2B** depicts another example of the results of transfection efficiency experiments using different cellular adhesion related agents (HLA, CD46, and CD54) in experiments using HepG2 cells.

As can be seen from the results, CD49 family antibodies caused significant transfection, as seen in the case of fibronectin. Accordingly, it was demonstrated that cellular adhesion related agents have significantly enhanced transfection efficiency.

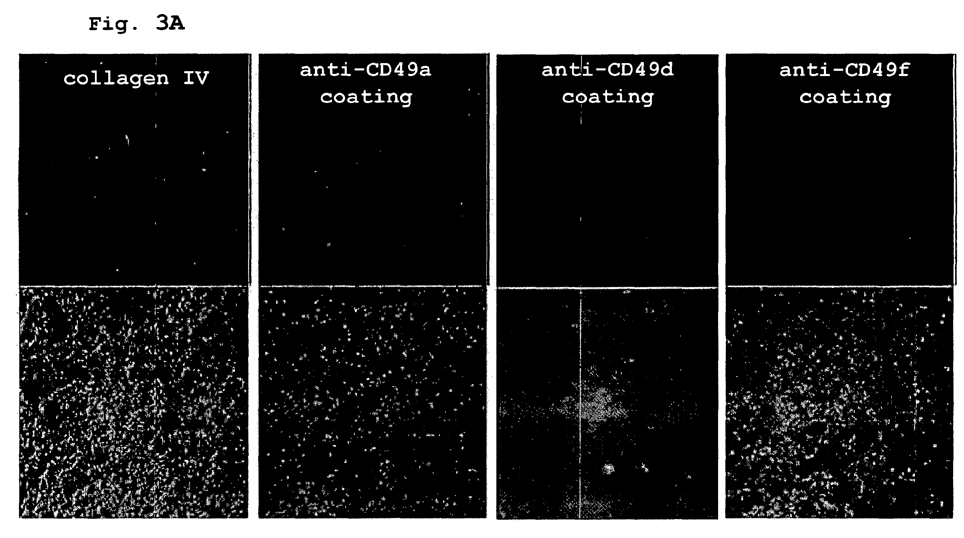

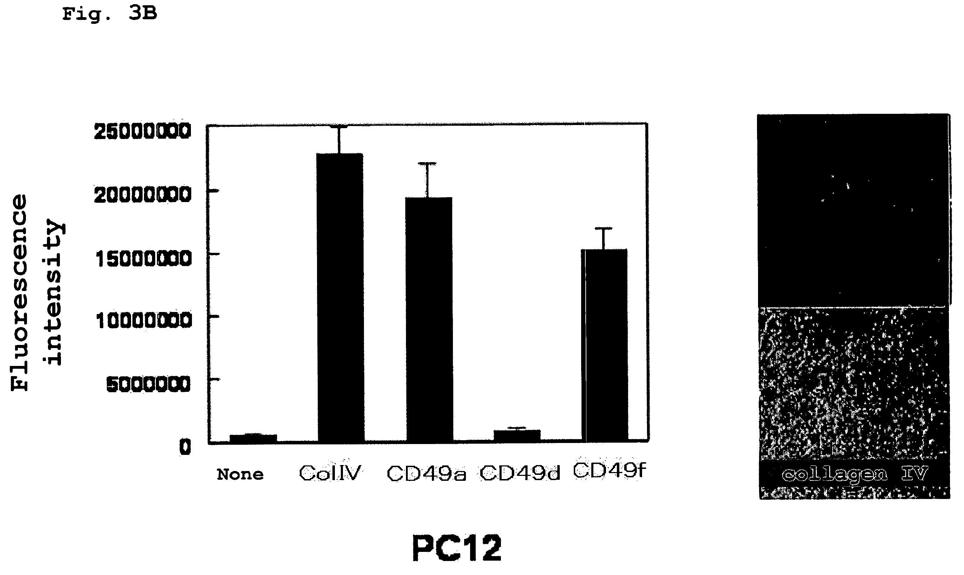

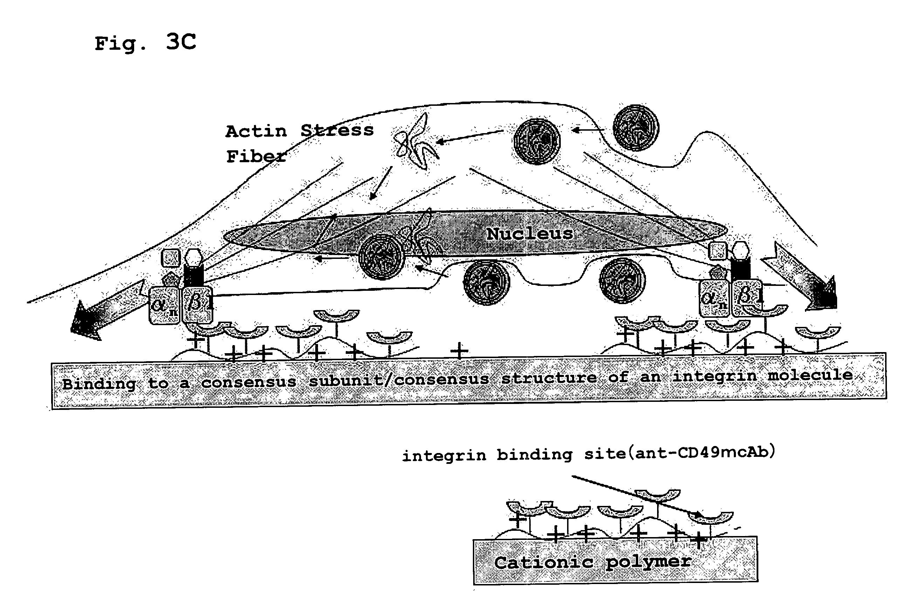

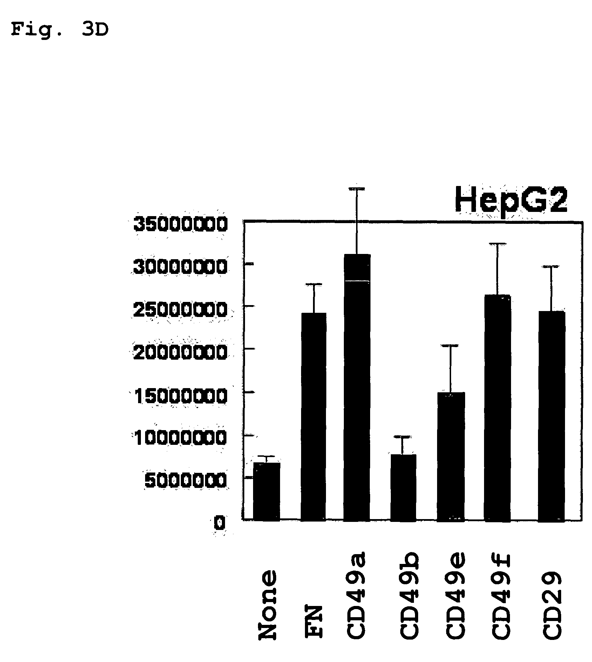

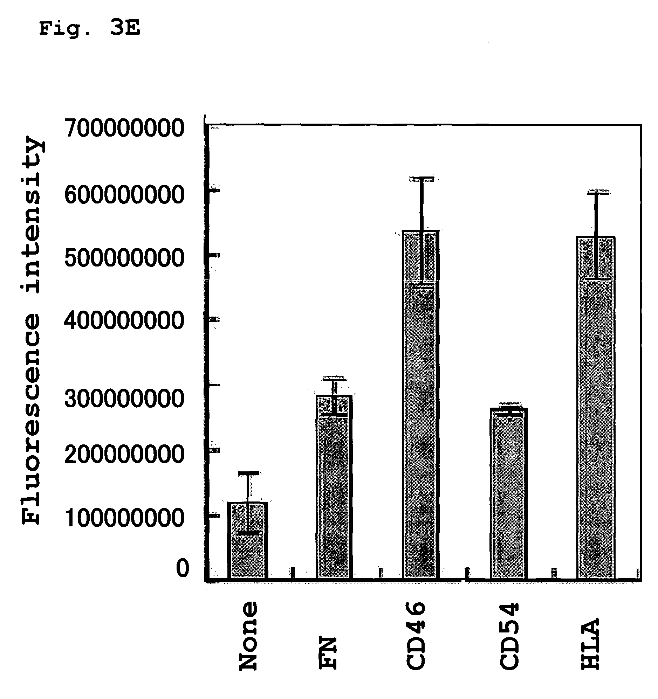

Figure **3A** shows the relationship between a variety of integrin receptors (CD49a, CD49d and CD49f) expressed on HepG2 cells with extracellular matrix proteins, and transfection efficiency on the surfaces coated with each extracellular matrix. Figures **3B-3E** show the change in cells after transfection in the case of coating with a variety of cellular adhesion related agents.

The addition of cellular adhesion related agents enhances the efficiency of transfection. As such, in addition to HEK293 cells, HeLa cells, and 3T3 cells, which are conventionally reported to be transfectable, transfection efficiency comparable to that of HeLa cells and 3T3 cells was achieved in HepG2 cells cells, which have been believed to be difficult to transfect. Such results have never been achieved by means of conventional transfection systems, and thus the claimed invention has allowed enhancement of transfection efficiency in substantially all cells. As such, it can be said that systems that permit transfection of all cells, which can practically be used, has been provided for the first time. Furthermore, by means of employing solid conditions, cross-contamination has been significantly reduced. Accordingly, it has been demonstrated that the present invention is an appropriate method for producing integrated bioarrays when using a solid support.

Furthermore, when using a variety of plates, plates with coating were found to have reduced contamination compared with those without coating, and the transfection efficiency thereof was also enhanced.

Furthermore, the transfection efficiency has been elucidated to be enhanced with increased concentrations of antibody. It is found, however, that the efficiency reaches plateau at a predetermined concentration.

### (EXAMPLE 4: Demonstration with PC12 cells)

PC12 cells, which are known as neuron-like cells, were used to observe whether or not differentiated cells attain effects according to the cellular adhesion related agents of the present invention.

According to Example 3, a variety of reagents and cells were prepared. PC12 cells, however, were prepared as follows:
PC12 (rat pheochromocytoma cells: ATCC CRL-1721) were cultured in a DMEM/10% calf serum (GIBCO) supplemented with L-glut and pen/strep. In order that the cell concentration be 10⁷ cells per 25ml, proliferating cells were provided. Square Petri dishes of 100x100x15mm or circular dishes of 100mm radius x 15mm were used for plating the cells on a slide. Transfection was carried out for about forty-eight hours to treat slides for immunological fluorescence.

### (RESULTS)

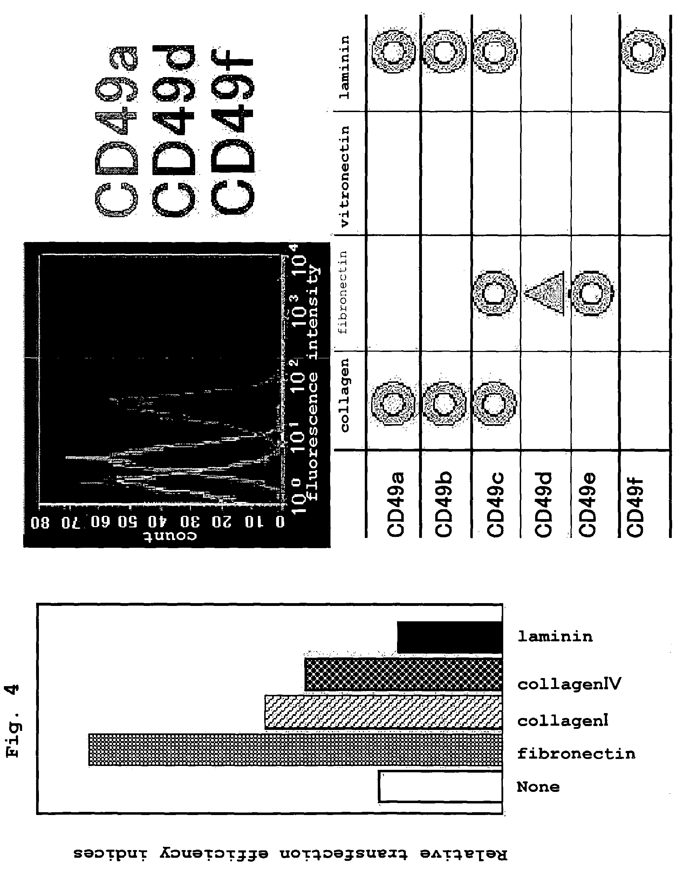

Results are shown in Figures **4-7.** Figure **4** depicts similar results to Figure **3A in** Example 3. As seen in the figure, it was elucidated that the cellular adhesion related agent according to the present invention enhances the transfection efficiency on PC12 cells, for which fibronectin has less effects.

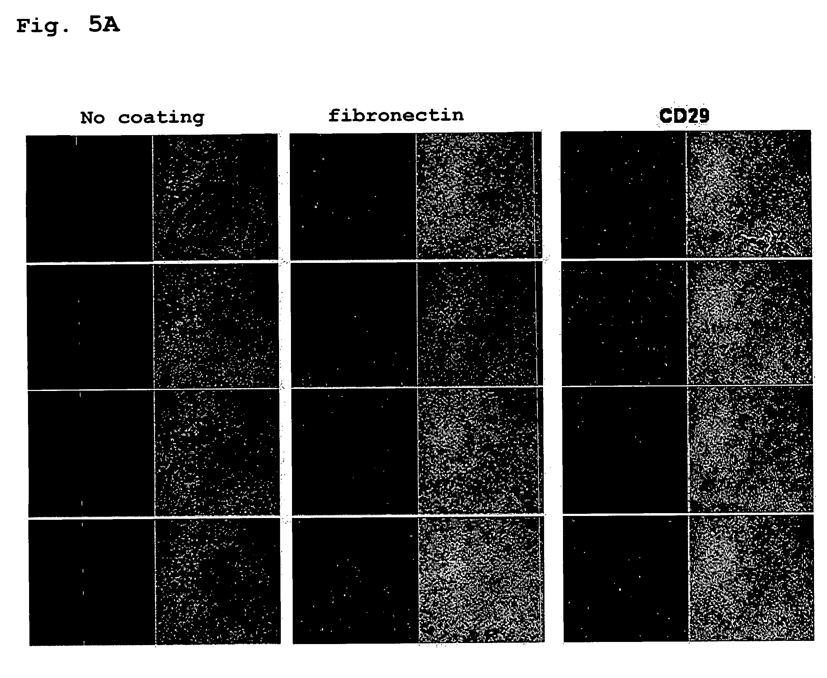

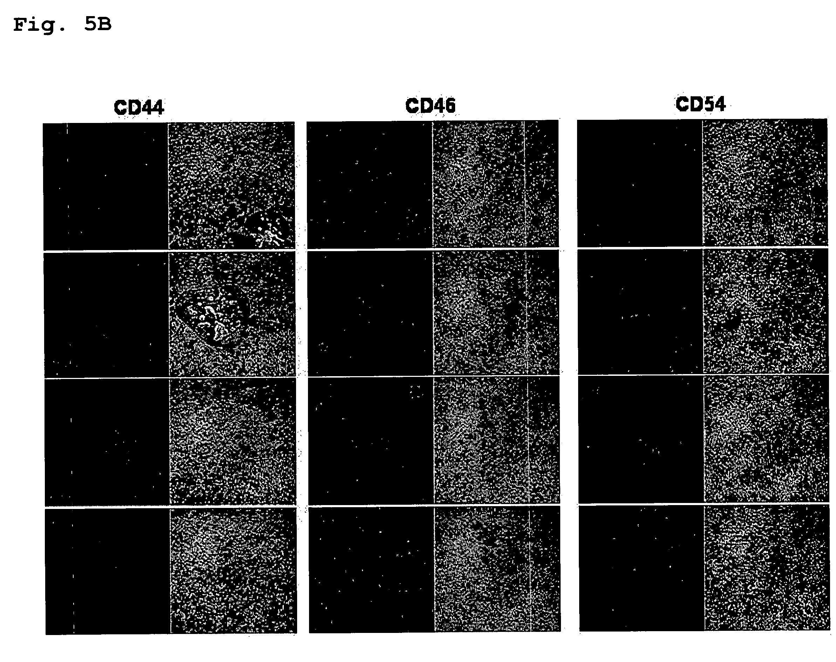

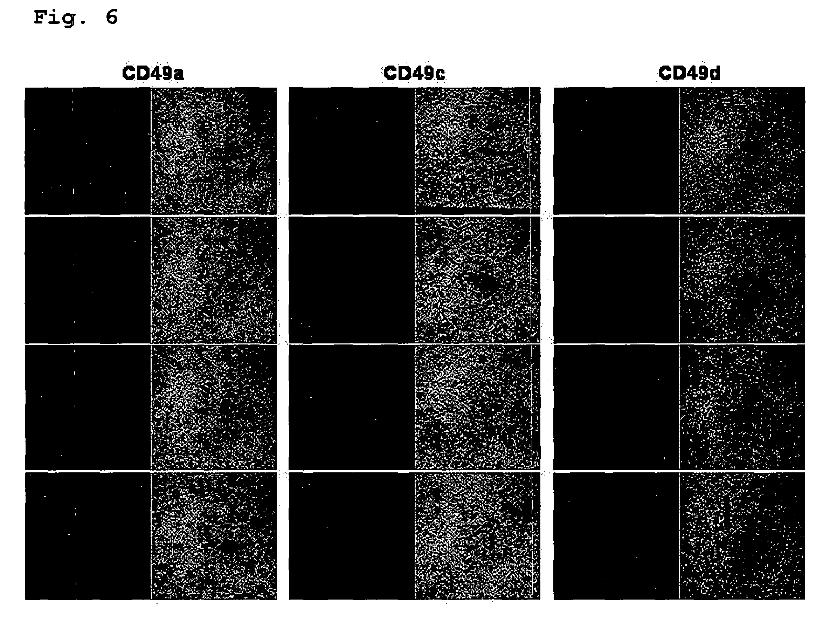

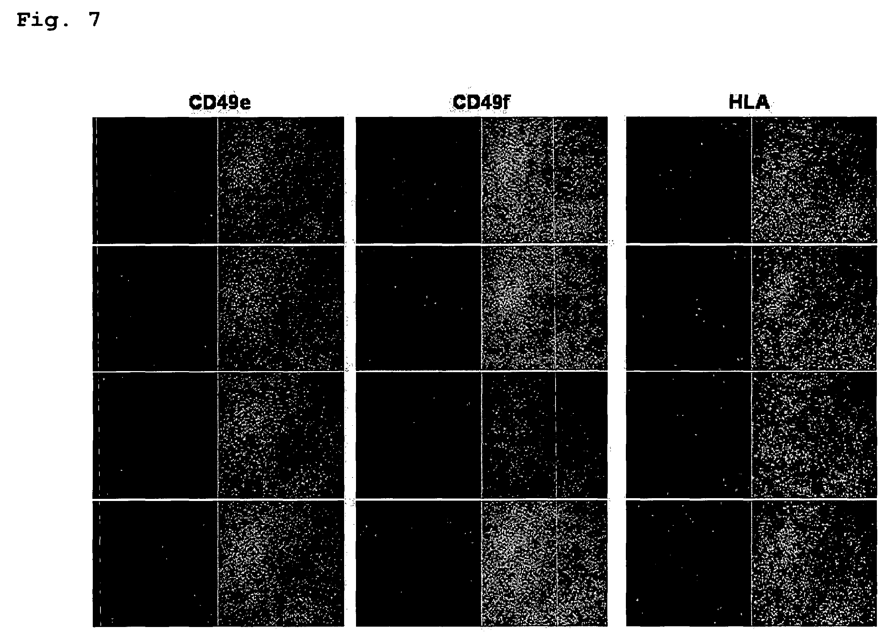

Figure **5A-B** depicts the state of cell adhesion. Figure **6** shows a series of photographs comparing the states of transfection. Figure **5A-B** depicts cell adhesion of PC12 cells in Example 4. It shows adhesion inhibition and transfection of PC12 cells on a Type IV collagen coated surface using CD antibodies. Type IV collagen was coated onto poly-L-lysine coated slides, and thereafter, PC12 cells which had been previously contacted with an antibody solution were seeded and transfected with Lipofectamine 2000 according to the conventional protocol for use in liquid phase. Figure **5A** shows that anti-CD49a antibody significantly inhibited the adhesion of PC12 cells onto Type IV collagen coated surfaces. An anti-CD49d antibody shows the similar transfection efficiency as the control without antibody, by exhibiting no inhibition of adhesion of PC12 cells. As seen from Figure **4,** PC12 cells do not express CD49d. Furthermore, CD49d is a receptor against fibronectin, and thus it is believed that the adhesion of Type IV collagen coated surface is not inhibited. That is, the existence of an antibody per se does not affect the transfection efficiency. Figure **5B** similarly shows the difference in transfection efficiency in the presence of an anti-CD49f antibody. The values shown indicate the dilution of the antibody stock solution used for contacting the PC12 cells. As clearly seen from the figure, transfection efficiency is reduced in a manner depending on the concentration of an antibody to be contacted. CD49f is a receptor to laminin, and thus it is believed that adhesion to Type IV collagen coated surfaces is not inhibited. As seen from Figure **4,** PC12 cells express CD49f, and the transfection efficiency is enhanced by laminin, and thus anti-CD49f allows enhancement of transfection efficiency thereof (see Figures **6** and **7).**

As seen from Figure **5,** transfection efficiency is reduced in a manner depending on the concentration of an antibody contacted thereto. CD49f is a receptor to laminin, and thus it is believed that adhesion to Type IV collagen coated surface is not inhibited. As seen from Figure **4,** PC12 cells express CD49f, and the transfection efficiency is enhanced by laminin, and thus anti-CD49f allows enhancement of transfection efficiency thereof (see Figures **6** and **7).** Therefore, as shown in Figure **7,** it was demonstrated that anti-CD49f antibody was used to enhance transfection efficiency.

As such, the inhibition of cellular adhesion and the enhancement of transfection is directly related. Such relationship has not been known conventionally, and thus the present invention shows that by inhibiting the cellular adhesion, it was possible to enhance the transfection efficiency, and thus it is possible to enhance transfection efficiency in any cells by inhibiting cellular adhesion.

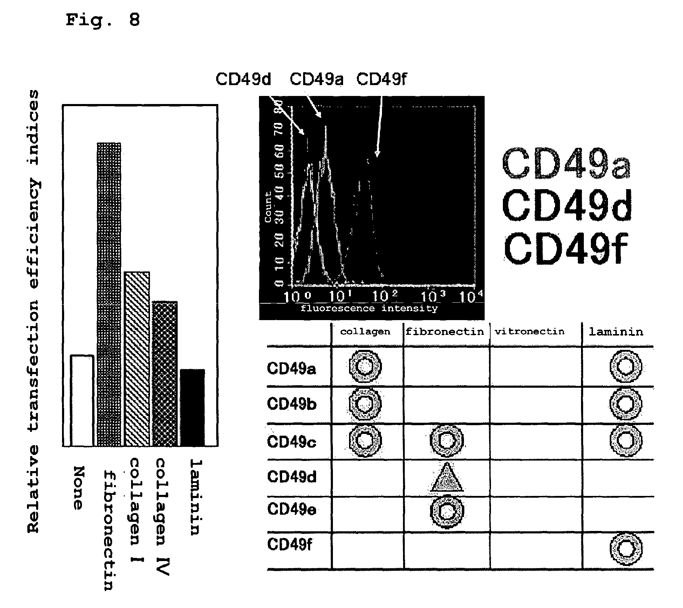

Figure **7** shows the effects of coating of cellular adhesion related agent on a support. Coating with collagen type IV appears to enhance transfection efficiency. The present inventors developed a complex-salt system, which could be used to achieve solid phase transfection which makes it possible to obtain high transfection efficiency with various cell lines (HepG2) and special localization in high-density arrays. Figure **8** depicts the outlines of solid transfection using a cellular adhesion related agent.

It was demonstrated that solid phase transfection can be used to achieve a "transfection patch" capable of being used for in vivo gene delivery and a solid phase transfection array (SPTA) for high-throughput genetic function research on HepG2 cells and PC12 cells.

Although a number of standard techniques are available for transfecting mammalian cells, it is known that it is inconvenient and difficult to introduce genetic material into cell lines such as HepG2 and PC12 and the like, as compared with cell lines such as HEK293, HeLa, and the like. Conventional viral vector delivery and electroporation techniques are each important. However, these techniques have the following inconveniences: potential toxicity (for the virus technique); difficulty in high-throughput analysis at the genomic scale; and limited applications in vivo studies (for electroporation).

The present inventors have developed a solid phase support fixed system which can be easily fixed to a solid phase support and has sustained-release capability and cell affinity, whereby most of the above-described drawbacks could be overcome.

The present inventors used their microprinting technique to fix a mixture of a selected genetic material, a transfection reagent, an appropriate cell adhesion molecule, and a salt onto a solid support. By culturing cells on a support having such a mixture fixed thereonto, the gene contained in the mixture was able to be taken in by the cultured cells. As a result, it became possible to allow support-adherent cells to take in DNA spatially separated therefrom.

As a result of this example, several important effects were achieved: high transfection efficiency (thereby making it possible to study a group of cells having a statistically significant scale) ; low cross contamination between regions having different DNA molecules (thereby making it possible to study the effects of different genes separately); the extended survival of transfected cells; high-throughput, compatibility and simple detection procedure. SPTA having these features serves as an appropriate basis for further studies.

To achieve the above-described objects, the present inventors studied different cell lines (HepG2 cells and PC12 cells), which are not efficiently transfected using fibronectin, as described above, with both our methodology including the present method (transfection in a solid phase system) and conventional liquid-phase transfection under a series of transfection conditions.

Transfection efficiency: transfection efficiency was determined as total fluorescence intensity per unit area. The results of liquid phase optimal to cell lines used were obtained. Next, these efficient transfection reagents were used to optimize a solid phase protocol. Several tendencies were observed. For cell lines which are readily transfectable (e.g., HEK293, HeLa, NIH3T3, etc.), the transfection efficiency observed in the solid phase protocol was slightly superior to, but essentially similar to, that of the standard liquid phase protocol.

However, for cells which are difficult to transfect (e.g., PC12 cells, HepG2 cells, etc.), we observed that transfection efficiency was increased up to 100 fold, while the features of the cells were retained under conditions optimized to the SPTA methodology. In the preliminary experimental results, HeLa cells show essentially high transfection, as described above.

The glass coating used is crucial for the achievement of high transfection efficiency on chips. It was found that PLL provided best results both for transfection efficiency and cross contamination. When a cellular adhesion related agent coating was not used, few transfectants were observed (all the other experimental conditions remained unchanged). Although not completely established, the cellular adhesion related agent probably plays a role in limiting the time which permits introduction of DNA.

Low cross contamination: apart from the higher transfection efficiency observed in the SPTA protocol, an important advantage of the present technique is to achieve an array of separated cells, in which selected genes are expressed in separate positions. The present inventors printed JetPEI and two different reporter genes mixed with fibronectin onto glass surface coated with fibronectin. The resultant transfection chip was subjected to appropriate cell culture. Expressed GFP and RFP were localized in regions, in which corresponding cDNA had been spotted, under the experimental conditions which had been found to be best. Substantially no cross contamination was observed.

This established technique is of particular importance in the context of cost-effective high-throughput gene function screening. Indeed, the small amounts of transfection reagent and DNA required, as well as the possible automatization of the entire process (from plasmid isolation to detection) increase the utility of the above presented method.

In conclusion, the present inventors successfully realized a PC12 cell and human mesenchymal stem cell(hMSC) transfection array in a system using complex-salt. With this technique, it will be possible to achieve high-throughput studies using the solid phase transfection, such as the elucidation of the genetic mechanism for differentiation of pluripotent stem cells. It was also elucidated that the detailed mechanism of the solid phase transfection as well as methodologies for the use of this technology for high throughput, real time gene expression monitoring can be applied for various purposes.

### (Example 5: Demonstration in a human mesenchymal stem cell)

With respect to transfection, the solid transfection method as described in Example 3 was used to conduct the present Example.

The cells were prepared as follows: hMSCs (human mesenchymal stem cells, PT-2501, Cambrex BioScience Walkersville, Inc. , MD) were used. In the case of human MSC cells, the cells were maintained in human mesenchymal cells basal medium (MSCGM BulletKit PT-3001, a commercially available medium from Cambrex BioScience Walkersville, Inc., MD) .

As in the results of Example 4, the present inventors have realized a transfection array of human mesenchymal stem cells in a system using a cellular adhesion related agent. This allows high throughput study in a variety of research methods using solid transfection, such as clarification of the genetic mechanisms controlling the differentiation of pluripotent stem cells. It was elucidated that the detailed machinery of solid transfection, and methodology relating to the use of technology of high through put real time gene expression monitoring, are applicable to a variety of purposes.

### (Example 6: Demonstration in a human neuroblastoma cell line)

SHSY5Y cells(human neuroblastoma: ATCC CRL-2266) were used to demonstrate the transfection efficiency of a neuroblastoma cell line.

Transfection was conducted according to Example 3. The cells were prepared by culture in a DMEM/10% FBS supplemented with L-glutamine and penicillin/streptomycin.

As in the results of Example 4, the present inventors have realized a transfection array of human mesenchymal stem cells in a system using a cellular adhesion related agent. This allows high throughput study in a variety of research methods using solid transfection, such as the clarification of the genetic mechanisms controlling the differentiation of pluripotent stem cells. It was elucidated that detailed machinery of solid transfection, and methodology relating to the use of technology of high through put real time gene expression monitoring, are applicable to a variety of purposes.

Although certain preferred embodiments have been described herein, it is not intended that such embodiments be construed as limitations of the scope of the invention except as set forth in the appended claims. Various other modifications and equivalents will be apparent to and can be readily made by those skilled in the art, after reading the description herein, without departing from the scope and spirit of this invention. All patents, published patent applications and publications cited herein are incorporated by reference as if set forth fully herein.

### INDUSTRIAL APPLICABILITY

According to the present invention, transfection efficiency can be increased either in a solid phase or in a liquid phase. The reagent for increasing transfection efficiency is useful for transfection in, particularly, solid phases. Accordingly, it is useful in any field using genetic engineering.

## Claims

1. A composition for enhancing the introduction efficiency of a target substance into a cell, comprising a cellular adhesion related agent.

2. A composition for enhancing the introduction efficiency of a target substance into a cell according to claim 1 wherein the cellular adhesion related agent comprises an interaction substance interacting with a cellular adhesion molecule.

3. A composition according to claim 2, wherein the cellular adhesion molecule is an extracellular matrix.

4. A composition according to claim 2, wherein the cellular adhesion molecule is an integrin receptor.

5. A composition according to claim 2, wherein the cellular adhesion molecule comprises an RGD molecule.

6. A composition according to claim 2, wherein the interaction molecule raises an antigen-antibody reaction with a partner of the cellular adhesion molecule.

7. A composition according to claim 2, wherein the interaction molecule is an antibody or a derivative thereof.

8. A composition according to claim 2, wherein the interaction molecule is a monoclonal or polyclonal antibody.

9. A composition according to claim 2, wherein the interaction molecule comprises an antibody selected from the group consisting of an anti-CD49a antibody, an anti-CD49b antibody, an anti-CD49c antibody, an anti-CD49e antibody, and an anti-CD49f antibody.

10. A composition according to claim 1, wherein the target substance comprises a genetic material.

11. A composition according to claim 1, wherein the target substance comprises a nucleic acid molecule.

12. A composition according to claim 1, wherein the target substance comprises DNA.

13. A composition according to claim 4, wherein the integerin receptor is selected from the group consisting of CD49a, CD49b, CD49c, CD49d, CD49e, CD49f and CD29.

14. A composition according to claim 4, wherein the integrin receptor is selected from the group consisting of CD29, CD49a, CD49c, Cd49d, CD49e and CD49f.

15. A composition according to claim 4, wherein the intergrin receptor interacts with a molecule selected from the group consisting of collagen, fibronectin, vitronectin and laminin.

16. A composition according to claim 1, wherein the cell comprises at least one cell selected from the group consisting of a stem cell and a differentiated cell.

17. A composition according to claim 1, wherein the cellular adhesion molecule is specifically expressed in the cell.

18. A composition according to claim 1, wherein the target substance is a genetic material and the composition further comprises a gene introduction reagent.

19. A composition according to claim 18, wherein the gene introduction reagent is selected from the group consisting of a cationic macromolecule, cationic lipid and calcium phosphate.

20. A composition according to claim 1, further comprising a particle.

21. A composition according to claim 20, wherein the particle comprises a gold colloid.

22. A composition according to claim 1 further comprising a salt.

23. A composition according to claim 22, wherein the salt is selected from the group consisting of salts comprised in a buffer and salts comprised in media.

24. A kit for enhancing gene introduction efficiency, comprising:
(a) a cellular adhesion related agent; and
(b) a gene introduction reagent.

25. A composition for introducing a target material to a cell, comprising:
(A) a target material; and
(B) a cellular adhesion related agent.

26. A composition according to claim 25, wherein the target material comprises a substance selected from the group consisting of DNA, RNA, polypeptide, sugar and a complex thereof.

27. A composition according to claim 25, wherein the target material comprises a DNA encoding a gene sequence to be transfected into the cell.

28. A composition according to claim 25 further comprising a gene introduction reagent.

29. A composition according to claim 25, wherein the cellular adhesion related agent comprises an interaction substance interacting with a cellular adhesion molecule.

30. A composition according to claim 25, wherein the cellular adhesion related agent comprises an antibody to a cellular adhesion molecule.

31. A composition according to claim 25 which is present as a liquid phase.

32. A composition according to claim 25 which is present as a solid phase.

33. A device for enhancing gene introduction efficiency of a target molecule into a cell, comprising:
(a) a target molecule; and
(b) a cellular adhesion related agent,
wherein the cellular adhesion related agent is immobilized onto a support.

34. A device according to claim 33, wherein the target substance comprises a substance selected from the group consisting of DNA, RNA, polypeptide, sugar and a complex thereof.

35. A device according to claim 33, wherein the target substance comprises a DNA encoding a gene sequence for the purpose of gene expression.

36. A device according to claim 33, further comprising a gene introduction reagent.

37. A device according to claim 36, wherein the cellular adhesion related agent comprises an interaction substance interacting with a cellular adhesion molecule.

38. A device according to claim 36, wherein the cellular adhesion related agent comprises an antibody against a cellular adhesion molecule.

39. A device according to claim 36, wherein the support is selected from the group consisting of a plate, a microwell plate, a tip, a slide glass, a film, a bead and metal.

40. A device according to claim 36, wherein the support is coated with a coating agent.

41. A device according to claim 40, wherein the coating agent comprises a substance selected from the group consisting of poly-L-lysine, silane, MAS, hydrophobic fluorine resin and metal.

42. A method for enhancing the introduction efficiency of a target substance into a cell, comprising the steps of:
A) providing a target substance;
B) providing a cellular adhesion related agent; and
C) contacting the target substance and the cellular adhesion related substance with the cell.

43. A method according to claim 42, wherein the target material comprises a substance selected from the group consisting of DNA, RNA, polypeptide, sugar and a complex thereof.

44. A method according to claim 43, wherein the target material comprises a DNA encoding a gene sequence to be transfected in the cell.

45. A method according to claim 42, further comprising a gene introduction reagent.

46. A method according to claim 42, wherein the cellular adhesion related agent comprises an interaction substance interacting with a cellular adhesion molecule.

47. A method according to claim 42, wherein the cellular adhesion related agent comprises an antibody to a cellular adhesion molecule.

48. A method according to claim 46, wherein the cellular adhesion molecule is an extracellular matrix molecule.

49. A method according to claim 42, wherein the method is conducted in a liquid phase.

50. A method according to claim 42, wherein the method is conducted in a solid phase.

51. A method for enhancing the introduction efficiency of a target substance into a cell, comprising the steps of:
I) immobilizing a composition comprising
A) a target substance, and
B)a cellular adhesion molecule onto a support; and
II) contacting a cell to the composition on the support.

52. A method according to claim 51, further comprising the step of providing a gene introduction reagent, said gene introduction reagent being contacted with the cell.

53. A method according to claim 52, further comprising the step of forming a complex between the target substance and a gene introduction reagent after the provision thereof, wherein thereafter the cellular adhesion related agent is provided.

54. A method according to claim 51, wherein the cellular adhesion related agent comprises an interaction substance interacting with a cellular adhesion molecule.


SEQUENCE LISTING

<110>  National Institute of Advanced Industrial Science and Technology,
       Masato MIYAKE, Tomohiro YOSHIKAWA, Eiichiro UCHIMURA, Jun MIYAKE

<120>  Composition and method for elevating gene transfer efficiency

<130>  AI010PCT

<150>  JP 2004-22315
<151>  2004-01-29

<160>  14

<170>  PatentIn version 3.1

<210>  1
<211>  1929
<212>  DNA
<213>  Homo sapiens

<220>
<221>  CDS
<222>  (1)..(1929)
<223>  fibronectin 1


<400>  1
atg ctt agg ggt ccg ggg ccc ggg ctg ctg ctg ctg gcc gtc cag tgc        48
Met Leu Arg Gly Pro Gly Pro Gly Leu Leu Leu Leu Ala Val Gln Cys
1               5                   10                  15

ctg ggg aca gcg gtg ccc tcc acg gga gcc tcg aag agc aag agg cag        96
Leu Gly Thr Ala Val Pro Ser Thr Gly Ala Ser Lys Ser Lys Arg Gln
            20                  25                  30

gct cag caa atg gtt cag ccc cag tcc ccg gtg gct gtc agt caa agc       144
Ala Gln Gln Met Val Gln Pro Gln Ser Pro Val Ala Val Ser Gln Ser
        35                  40                  45

aag ccc ggt tgt tat gac aat gga aaa cac tat cag ata aat caa cag       192
Lys Pro Gly Cys Tyr Asp Asn Gly Lys His Tyr Gln Ile Asn Gln Gln
    50                  55                  60

tgg gag cgg acc tac cta ggc aat gcg ttg gtt tgt act tgt tat gga       240
Trp Glu Arg Thr Tyr Leu Gly Asn Ala Leu Val Cys Thr Cys Tyr Gly
65                  70                  75                  80

gga agc cga ggt ttt aac tgc gag agt aaa cct gaa gct gaa gag act       288
Gly Ser Arg Gly Phe Asn Cys Glu Ser Lys Pro Glu Ala Glu Glu Thr
                85                  90                  95

tgc ttt gac aag tac act ggg aac act tac cga gtg ggt gac act tat       336
Cys Phe Asp Lys Tyr Thr Gly Asn Thr Tyr Arg Val Gly Asp Thr Tyr
            100                 105                 110

gag cgt cct aaa gac tcc atg atc tgg gac tgt acc tgc atc ggg gct       384
Glu Arg Pro Lys Asp Ser Met Ile Trp Asp Cys Thr Cys Ile Gly Ala
        115                 120                 125

ggg cga ggg aga ata agc tgt acc atc gca aac cgc tgc cat gaa ggg       432
Gly Arg Gly Arg Ile Ser Cys Thr Ile Ala Asn Arg Cys His Glu Gly
    130                 135                 140

ggt cag tcc tac aag att ggt gac acc tgg agg aga cca cat gag act       480
Gly Gln Ser Tyr Lys Ile Gly Asp Thr Trp Arg Arg Pro His Glu Thr
145                 150                 155                 160

ggt ggt tac atg tta gag tgt gtg tgt ctt ggt aat gga aaa gga gaa 528
Gly Gly Tyr Met Leu Glu Cys Val Cys Leu Gly Asn Gly Lys Gly Glu
                165                 170                 175

tgg acc tgc aag ccc ata gct gag aag tgt ttt gat cat gct gct ggg 576
Trp Thr Cys Lys Pro Ile Ala Glu Lys Cys Phe Asp His Ala Ala Gly
            180                 185                 190

act tcc tat gtg gtc gga gaa acg tgg gag aag ccc tac caa ggc tgg 624
Thr Ser Tyr Val Val Gly Glu Thr Trp Glu Lys Pro Tyr Gln Gly Trp
        195                 200                 205

atg atg gta gat tgt act tgc ctg gga gaa ggc agc gga cgc atc act 672
Met Met Val Asp Cys Thr Cys Leu Gly Glu Gly Ser Gly Arg Ile Thr
    210                 215                 220

tgc act tct aga aat aga tgc aac gat cag gac aca agg aca tcc tat 720
Cys Thr Ser Arg Asn Arg Cys Asn Asp Gln Asp Thr Arg Thr Ser Tyr
225                 230                 235                 240

aga att gga gac acc tgg agc aag aag gat aat cga gga aac ctg ctc 768
Arg Ile Gly Asp Thr Trp Ser Lys Lys Asp Asn Arg Gly Asn Leu Leu
                245                 250                 255

cag tgc atc tgc aca ggc aac ggc cga gga gag tgg aag tgt gag agg 816
Gln Cys Ile Cys Thr Gly Asn Gly Arg Gly Glu Trp Lys Cys Glu Arg
            260                 265                 270

cac acc tct gtg cag acc aca tcg agc gga tct ggc ccc ttc acc gat 864
His Thr Ser Val Gln Thr Thr Ser Ser Gly Ser Gly Pro Phe Thr Asp
        275                 280                 285

gtt cgt gca gct gtt tac caa ccg cag cct cac ccc cag cct cct ccc 912
Val Arg Ala Ala Val Tyr Gln Pro Gln Pro His Pro Gln Pro Pro Pro
    290                 295                 300

tat ggc cac tgt gtc aca gac agt ggt gtg gtc tac tct gtg ggg atg 960
Tyr Gly His Cys Val Thr Asp Ser Gly Val Val Tyr Ser Val Gly Met
305                 310                 315                 320

cag tgg ctg aag aca caa gga aat aag caa atg ctt tgc acg tgc ctg 1008
Gln Trp Leu Lys Thr Gln Gly Asn Lys Gln Met Leu Cys Thr Cys Leu
                325                 330                 335

ggc aac gga gtc agc tgc caa gag aca gct gta acc cag act tac ggt 1056
Gly Asn Gly Val Ser Cys Gln Glu Thr Ala Val Thr Gln Thr Tyr Gly
            340                 345                 350

ggc aac tca aat gga gag cca tgt gtc tta cca ttc acc tac aat ggc 1104
Gly Asn Ser Asn Gly Glu Pro Cys Val Leu Pro Phe Thr Tyr Asn Gly
        355                 360                 365

agg acg gac agc aca act tcg aat tat gag cag gac cag aaa tac tct 1152
Arg Thr Asp Ser Thr Thr Ser Asn Tyr Glu Gln Asp Gln Lys Tyr Ser
    370                 375                 380

ttc tgc aca gac cac act gtt ttg gtt cag act cga gga gga aat tcc 1200
Phe Cys Thr Asp His Thr Val Leu Val Gln Thr Arg Gly Gly Asn Ser
385                 390                 395                 400

aat ggt gcc ttg tgc cac ttc ccc ttc cta tac aac aac cac aat tac 1248
Asn Gly Ala Leu Cys His Phe Pro Phe Leu Tyr Asn Asn His Asn Tyr
                405                 410                 415

act gat tgc act tct gag ggc aga aga gac aac atg aag tgg tgt ggg 1296
Thr Asp Cys Thr Ser Glu Gly Arg Arg Asp Asn Met Lys Trp Cys Gly
            420                 425                 430

acc aca cag aac tat gat gcc gac cag aag ttt ggg ttc tgc ccc atg 1344
Thr Thr Gln Asn Tyr Asp Ala Asp Gln Lys Phe Gly Phe Cys Pro Met
        435                 440                 445

gct gcc cac gag gaa atc tgc aca acc aat gaa ggg gtc atg tac cgc 1392
Ala Ala His Glu Glu Ile Cys Thr Thr Asn Glu Gly Val Met Tyr Arg
    450                 455                 460

att gga gat cag tgg gat aag cag cat gac atg ggt cac atg atg agg 1440
Ile Gly Asp Gln Trp Asp Lys Gln His Asp Met Gly His Met Met Arg
465                 470                 475                 480

tgc acg tgt gtt ggg aat ggt cgt ggg gaa tgg aca tgc att gcc tac 1488
Cys Thr Cys Val Gly Asn Gly Arg Gly Glu Trp Thr Cys Ile Ala Tyr
                485                 490                 495

tcg cag ctt cga gat cag tgc att gtt gat gac atc act tac aat gtg 1536
Ser Gln Leu Arg Asp Gln Cys Ile Val Asp Asp Ile Thr Tyr Asn Val
            500                 505                 510

aac gac aca ttc cac aag cgt cat gaa gag ggg cac atg ctg aac tgt 1584
Asn Asp Thr Phe His Lys Arg His Glu Glu Gly His Met Leu Asn Cys
        515                 520                 525

aca tgc ttc ggt cag ggt cgg ggc agg tgg aag tgt gat ccc gtc gac 1632
Thr Cys Phe Gly Gln Gly Arg Gly Arg Trp Lys Cys Asp Pro Val Asp
    530                 535                 540

caa tgc cag gat tca gag act ggg acg ttt tat caa att gga gat tca 1680
Gln Cys Gln Asp Ser Glu Thr Gly Thr Phe Tyr Gln Ile Gly Asp Ser
545                 550                 555                 560

tgg gag aag tat gtg cat ggt gtc aga tac cag tgc tac tgc tat ggc 1728
Trp Glu Lys Tyr Val His Gly Val Arg Tyr Gln Cys Tyr Cys Tyr Gly
                565                 570                 575

cgt ggc att ggg gag tgg cat tgc caa cct tta cag acc tat cca agc 1776
Arg Gly Ile Gly Glu Trp His Cys Gln Pro Leu Gln Thr Tyr Pro Ser
            580                 585                 590

tca agt ggt cct gtc gaa gta ttt atc act gag act ccg agt cag ccc 1824
Ser Ser Gly Pro Val Glu Val Phe Ile Thr Glu Thr Pro Ser Gln Pro
        595                 600                 605

aac tcc cac ccc atc cag tgg aat gca cca cag cca tct cac att tcc 1872
Asn Ser His Pro Ile Gln Trp Asn Ala Pro Gln Pro Ser His Ile Ser
    610                 615                 620

aag tac att ctc agg tgg aga cct gtg agt atc cca ccc aga aac ctt 1920
Lys Tyr Ile Leu Arg Trp Arg Pro Val Ser Ile Pro Pro Arg Asn Leu
625                 630                 635                 640

gga tac tga 1929
Gly Tyr

<210> 2
<211> 642
<212> PRT
<213> Homo sapiens

<400> 2

Met Leu Arg Gly Pro Gly Pro Gly Leu Leu Leu Leu Ala Val Gln Cys
1               5                   10                  15

Leu Gly Thr Ala Val Pro Ser Thr Gly Ala Ser Lys Ser Lys Arg Gln
            20                  25                  30

Ala Gln Gln Met Val Gln Pro Gln Ser Pro Val Ala Val Ser Gln Ser
        35                  40                  45

Lys Pro Gly Cys Tyr Asp Asn Gly Lys His Tyr Gln Ile Asn Gln Gln
    50                  55                  60

Trp Glu Arg Thr Tyr Leu Gly Asn Ala Leu Val Cys Thr Cys Tyr Gly
65                  70                  75                  80

Gly Ser Arg Gly Phe Asn Cys Glu Ser Lys Pro Glu Ala Glu Glu Thr
                85                  90                  95

Cys Phe Asp Lys Tyr Thr Gly Asn Thr Tyr Arg Val Gly Asp Thr Tyr
            100                 105                 110

Glu Arg Pro Lys Asp Ser Met Ile Trp Asp Cys Thr Cys Ile Gly Ala
        115                 120                 125

Gly Arg Gly Arg Ile Ser Cys Thr Ile Ala Asn Arg Cys His Glu Gly
    130                 135                 140

Gly Gln Ser Tyr Lys Ile Gly Asp Thr Trp Arg Arg Pro His Glu Thr
145                 150                 155                 160

Gly Gly Tyr Met Leu Glu Cys Val Cys Leu Gly Asn Gly Lys Gly Glu
                165                 170                 175

Trp Thr Cys Lys Pro Ile Ala Glu Lys Cys Phe Asp His Ala Ala Gly
            180                 185                 190

Thr Ser Tyr Val Val Gly Glu Thr Trp Glu Lys Pro Tyr Gln Gly Trp
        195                 200                 205

Met Met Val Asp Cys Thr Cys Leu Gly Glu Gly Ser Gly Arg Ile Thr
    210                 215                 220

Cys Thr Ser Arg Asn Arg Cys Asn Asp Gln Asp Thr Arg Thr Ser Tyr
225                 230                 235                 240

Arg Ile Gly Asp Thr Trp Ser Lys Lys Asp Asn Arg Gly Asn Leu Leu
                245                 250                 255

Gln Cys Ile Cys Thr Gly Asn Gly Arg Gly Glu Trp Lys Cys Glu Arg
            260                 265                 270

His Thr Ser Val Gln Thr Thr Ser Ser Gly Ser Gly Pro Phe Thr Asp
        275                 280                 285

Val Arg Ala Ala Val Tyr Gln Pro Gln Pro His Pro Gln Pro Pro Pro
    290                 295                 300

Tyr Gly His Cys Val Thr Asp Ser Gly Val Val Tyr Ser Val Gly Met
305                 310                 315                 320

Gln Trp Leu Lys Thr Gln Gly Asn Lys Gln Met Leu Cys Thr Cys Leu
                325                 330                 335

Gly Asn Gly Val Ser Cys Gln Glu Thr Ala Val Thr Gln Thr Tyr Gly
            340                 345                 350

Gly Asn Ser Asn Gly Glu Pro Cys Val Leu Pro Phe Thr Tyr Asn Gly
        355                 360                 365

Arg Thr Asp Ser Thr Thr Ser Asn Tyr Glu Gln Asp Gln Lys Tyr Ser
    370                 375                 380

Phe Cys Thr Asp His Thr Val Leu Val Gln Thr Arg Gly Gly Asn Ser
385                 390                 395                 400

Asn Gly Ala Leu Cys His Phe Pro Phe Leu Tyr Asn Asn His Asn Tyr
                405                 410                 415

Thr Asp Cys Thr Ser Glu Gly Arg Arg Asp Asn Met Lys Trp Cys Gly
            420                 425                 430

Thr Thr Gln Asn Tyr Asp Ala Asp Gln Lys Phe Gly Phe Cys Pro Met
        435                 440                 445

Ala Ala His Glu Glu Ile Cys Thr Thr Asn Glu Gly Val Met Tyr Arg
    450                 455                 460

Ile Gly Asp Gln Trp Asp Lys Gln His Asp Met Gly His Met Met Arg
465                 470                 475                 480

Cys Thr Cys Val Gly Asn Gly Arg Gly Glu Trp Thr Cys Ile Ala Tyr
                485                 490                 495

Ser Gln Leu Arg Asp Gln Cys Ile Val Asp Asp Ile Thr Tyr Asn Val
            500                 505                 510

Asn Asp Thr Phe His Lys Arg His Glu Glu Gly His Met Leu Asn Cys
        515                 520                 525

Thr Cys Phe Gly Gln Gly Arg Gly Arg Trp Lys Cys Asp Pro Val Asp
    530                 535                 540

Gln Cys Gln Asp Ser Glu Thr Gly Thr Phe Tyr Gln Ile Gly Asp Ser
545                 550                 555                 560

Trp Glu Lys Tyr Val His Gly Val Arg Tyr Gln Cys Tyr Cys Tyr Gly
                565                 570                 575

Arg Gly Ile Gly Glu Trp His Cys Gln Pro Leu Gln Thr Tyr Pro Ser
            580                 585                 590

Ser Ser Gly Pro Val Glu Val Phe Ile Thr Glu Thr Pro Ser Gln Pro
        595                 600                 605

Asn Ser His Pro Ile Gln Trp Asn Ala Pro Gln Pro Ser His Ile Ser
    610                 615                 620

Lys Tyr Ile Leu Arg Trp Arg Pro Val Ser Ile Pro Pro Arg Asn Leu
625                 630                 635                 640

Gly Tyr

<210>  3
<211>  798
<212>  PRT
<213>  Homo sapiens

<400>  3

Met Asn Leu Gln Pro Ile Phe Trp Ile Gly Leu Ile Ser Ser Val Cys
1               5                   10                  15

Cys Val Phe Ala Gln Thr Asp Glu Asn Arg Cys Leu Lys Ala Asn Ala
            20                  25                  30

Lys Ser Cys Gly Glu Cys Ile Gln Ala Gly Pro Asn Cys Gly Trp Cys
        35                  40                  45

Thr Asn Ser Thr Phe Leu Gln Glu Gly Met Pro Thr Ser Ala Arg Cys
    50                  55                  60

Asp Asp Leu Glu Ala Leu Lys Lys Lys Gly Cys Pro Pro Asp Asp Ile
65                  70                  75                  80

Glu Asn Pro Arg Gly Ser Lys Asp Ile Lys Lys Asn Lys Asn Val Thr
                85                  90                  95

Asn Arg Ser Lys Gly Thr Ala Glu Lys Leu Lys Pro Glu Asp Ile Thr
            100                 105                 110

Gln Ile Gln Pro Gln Gln Leu Val Leu Arg Leu Arg Ser Gly Glu Pro
        115                 120                 125

Gln Thr Phe Thr Leu Lys Phe Lys Arg Ala Glu Asp Tyr Pro Ile Asp
    130                 135                 140

Leu Tyr Tyr Leu Met Asp Leu Ser Tyr Ser Met Lys Asp Asp Leu Glu
145                 150                 155                 160

Asn Val Lys Ser Leu Gly Thr Asp Leu Met Asn Glu Met Arg Arg Ile
                165                 170                 175

Thr Ser Asp Phe Arg Ile Gly Phe Gly Ser Phe Val Glu Lys Thr Val
            180                 185                 190

Met Pro Tyr Ile Ser Thr Thr Pro Ala Lys Leu Arg Asn Pro Cys Thr
        195                 200                 205

Ser Glu Gln Asn Cys Thr Ser Pro Phe Ser Tyr Lys Asn Val Leu Ser
    210                 215                 220

Leu Thr Asn Lys Gly Glu Val Phe Asn Glu Leu Val Gly Lys Gln Arg
225                 230                 235                 240

Ile Ser Gly Asn Leu Asp Ser Pro Glu Gly Gly Phe Asp Ala Ile Met
                245                 250                 255

Gln Val Ala Val Cys Gly Ser Leu Ile Gly Trp Arg Asn Val Thr Arg
            260                 265                 270

Leu Leu Val Phe Ser Thr Asp Ala Gly Phe His Phe Ala Gly Asp Gly
        275                 280                 285

Lys Leu Gly Gly Ile Val Leu Pro Asn Asp Gly Gln Cys His Leu Glu
    290                 295                 300

Asn Asn Met Tyr Thr Met Ser His Tyr Tyr Asp Tyr Pro Ser Ile Ala
305                 310                 315                 320

His Leu Val Gln Lys Leu Ser Glu Asn Asn Ile Gln Thr Ile Phe Ala
                325                 330                 335

Val Thr Glu Glu Phe Gln Pro Val Tyr Lys Glu Leu Lys Asn Leu Ile
            340                 345                 350

Pro Lys Ser Ala Val Gly Thr Leu Ser Ala Asn Ser Ser Asn Val Ile
        355                 360                 365

Gln Leu Ile Ile Asp Ala Tyr Asn Ser Leu Ser Ser Glu Val Ile Leu
    370                 375                 380

Glu Asn Gly Lys Leu Ser Glu Gly Val Thr Ile Ser Tyr Lys Ser Tyr
385                 390                 395                 400

Cys Lys Asn Gly Val Asn Gly Thr Gly Glu Asn Gly Arg Lys Cys Ser
                405                 410                 415

Asn Ile Ser Ile Gly Asp Glu Val Gln Phe Glu Ile Ser Ile Thr Ser
            420                 425                 430

Asn Lys Cys Pro Lys Lys Asp Ser Asp Ser Phe Lys Ile Arg Pro Leu
        435                 440                 445

Gly Phe Thr Glu Glu Val Glu Val Ile Leu Gln Tyr Ile Cys Glu Cys
    450                 455                 460

Glu Cys Gln Ser Glu Gly Ile Pro Glu Ser Pro Lys Cys His Glu Gly
465                 470                 475                 480

Asn Gly Thr Phe Glu Cys Gly Ala Cys Arg Cys Asn Glu Gly Arg Val
                485                 490                 495

Gly Arg His Cys Glu Cys Ser Thr Asp Glu Val Asn Ser Glu Asp Met
            500                 505                 510

Asp Ala Tyr Cys Arg Lys Glu Asn Ser Ser Glu Ile Cys Ser Asn Asn
        515                 520                 525

Gly Glu Cys Val Cys Gly Gln Cys Val Cys Arg Lys Arg Asp Asn Thr
    530                 535                 540

Asn Glu Ile Tyr Ser Gly Lys Phe Cys Glu Cys Asp Asn Phe Asn Cys
545                 550                 555                 560

Asp Arg Ser Asn Gly Leu Ile Cys Gly Gly Asn Gly Val Cys Lys Cys
                565                 570                 575

Arg Val Cys Glu Cys Asn Pro Asn Tyr Thr Gly Ser Ala Cys Asp Cys
            580                 585                 590

Ser Leu Asp Thr Ser Thr Cys Glu Ala Ser Asn Gly Gln Ile Cys Asn
        595                 600                 605

Gly Arg Gly Ile Cys Glu Cys Gly Val Cys Lys Cys Thr Asp Pro Lys
    610                 615                 620

Phe Gln Gly Gln Thr Cys Glu Met Cys Gln Thr Cys Leu Gly Val Cys
625                 630                 635                 640

Ala Glu His Lys Glu Cys Val Gln Cys Arg Ala Phe Asn Lys Gly Glu
                645                 650                 655

Lys Lys Asp Thr Cys Thr Gln Glu Cys Ser Tyr Phe Asn Ile Thr Lys
            660                 665                 670

Val Glu Ser Arg Asp Lys Leu Pro Gln Pro Val Gln Pro Asp Pro Val
        675                 680                 685

Ser His Cys Lys Glu Lys Asp Val Asp Asp Cys Trp Phe Tyr Phe Thr
    690                 695                 700

Tyr Ser Val Asn Gly Asn Asn Glu Val Met Val His Val Val Glu Asn
705                 710                 715                 720

Pro Glu Cys Pro Thr Gly Pro Asp Ile Ile Pro Ile Val Ala Gly Val
                725                 730                 735

Val Ala Gly Ile Val Leu Ile Gly Leu Ala Leu Leu Leu Ile Trp Lys
            740                 745                 750

Leu Leu Met Ile Ile His Asp Arg Arg Glu Phe Ala Lys Phe Glu Lys
        755                 760                 765

Glu Lys Met Asn Ala Lys Trp Asp Thr Gly Glu Asn Pro Ile Tyr Lys
    770                 775                 780

Ser Ala Val Thr Thr Val Val Asn Pro Lys Tyr Glu Gly Lys
785                 790                 795

<210>  4
<211>  1151
<212>  PRT
<213>  Homo sapiens

<400>  4

Phe Asn Val Asp Val Lys Asn Ser Met Thr Phe Ser Gly Pro Val Glu
1               5                   10                  15

Asp Met Phe Gly Tyr Thr Val Gln Gln Tyr Glu Asn Glu Glu Gly Lys
            20                  25                  30

Trp Val Leu Ile Gly Ser Pro Leu Val Gly Gln Pro Lys Asn Arg Thr
        35                  40                  45

Gly Asp Val Tyr Lys Cys Pro Val Gly Arg Gly Glu Ser Leu Pro Cys
    50                  55                  60

Val Lys Leu Asp Leu Pro Val Asn Thr Ser Ile Pro Asn Val Thr Glu
65                  70                  75                  80

Val Lys Glu Asn Met Thr Phe Gly Ser Thr Leu Val Thr Asn Pro Asn
                85                  90                  95

Gly Gly Phe Leu Ala Cys Gly Pro Leu Tyr Ala Tyr Arg Cys Gly His
            100                 105                 110

Leu His Tyr Thr Thr Gly Ile Cys Ser Asp Val Ser Pro Thr Phe Gln
        115                 120                 125

Val Val Asn Ser Ile Ala Pro Val Gln Glu Cys Ser Thr Gln Leu Asp

130                 135                 140

Ile Val Ile Val Leu Asp Gly Ser Asn Ser Ile Tyr Pro Trp Asp Ser
145                 150                 155                 160

Val Thr Ala Phe Leu Asn Asp Leu Leu Lys Arg Met Asp Ile Gly Pro
                165                 170                 175

Lys Gln Thr Gln Val Gly Ile Val Gln Tyr Gly Glu Asn Val Thr His
            180                 185                 190

Glu Phe Asn Leu Asn Lys Tyr Ser Ser Thr Glu Glu Val Leu Val Ala
        195                 200                 205

Ala Lys Lys Ile Val Gln Arg Gly Gly Arg Gln Thr Met Thr Ala Leu
    210                 215                 220

Gly Thr Asp Thr Ala Arg Lys Glu Ala Phe Thr Glu Ala Arg Gly Ala
225                 230                 235                 240

Arg Arg Gly Val Lys Lys Val Met Val Ile Val Thr Asp Gly Glu Ser
                245                 250                 255

His Asp Asn His Arg Leu Lys Lys Val Ile Gln Asp Cys Glu Asp Glu
            260                 265                 270

Asn Ile Gln Arg Phe Ser Ile Ala Ile Leu Gly Ser Tyr Asn Arg Gly
        275                 280                 285

Asn Leu Ser Thr Glu Lys Phe Val Glu Glu Ile Lys Ser Ile Ala Ser
    290                 295                 300

Glu Pro Thr Glu Lys His Phe Phe Asn Val Ser Asp Glu Leu Ala Leu
305                 310                 315                 320

Val Thr Ile Val Lys Thr Leu Gly Glu Arg Ile Phe Ala Leu Glu Ala
                325                 330                 335

Thr Ala Asp Gln Ser Ala Ala Ser Phe Glu Met Glu Met Ser Gln Thr
            340                 345                 350

Gly Phe Ser Ala His Tyr Ser Gln Asp Trp Val Met Leu Gly Ala Val
        355                 360                 365

Gly Ala Tyr Asp Trp Asn Gly Thr Val Val Met Gln Lys Ala Ser Gln
    370                 375                 380

Ile Ile Ile Pro Arg Asn Thr Thr Phe Asn Val Glu Ser Thr Lys Lys
385                 390                 395                 400

Asn Glu Pro Leu Ala Ser Tyr Leu Gly Tyr Thr Val Asn Ser Ala Thr

405                                    410                                    415

Ala Ser Ser Gly Asp Val Leu Tyr Ile Ala Gly Gln Pro Arg Tyr Asn
            420                 425                 430

His Thr Gly Gln Val Ile Ile Tyr Arg Met Glu Asp Gly Asn Ile Lys
        435                 440                 445

Ile Leu Gln Thr Leu Ser Gly Glu Gln Ile Gly Ser Tyr Phe Gly Ser
    450                 455                 460

Ile Leu Thr Thr Thr Asp Ile Asp Lys Asp Ser Asn Thr Asp Ile Leu
465                 470                 475                 480

Leu Val Gly Ala Pro Met Tyr Met Gly Thr Glu Lys Glu Glu Gln Gly
                485                 490                 495

Lys Val Tyr Val Tyr Ala Leu Asn Gln Thr Arg Phe Glu Tyr Gln Met
            500                 505                 510

Ser Leu Glu Pro Ile Lys Gln Thr Cys Cys Ser Ser Arg Gln His Asn
        515                 520                 525

Ser Cys Thr Thr Glu Asn Lys Asn Glu Pro Cys Gly Ala Arg Phe Gly
    530                 535                 540

Thr Ala Ile Ala Ala Val Lys Asp Leu Asn Leu Asp Gly Phe Asn Asp
545                 550                 555                 560

Ile Val Ile Gly Ala Pro Leu Glu Asp Asp His Gly Gly Ala Val Tyr
                565                 570                 575

Ile Tyr His Gly Ser Gly Lys Thr Ile Arg Lys Glu Tyr Ala Gln Arg
            580                 585                 590

Ile Pro Ser Gly Gly Asp Gly Lys Thr Leu Lys Phe Phe Gly Gln Ser
        595                 600                 605

Ile His Gly Glu Met Asp Leu Asn Gly Asp Gly Leu Thr Asp Val Thr
    610                 615                 620

Ile Gly Gly Leu Gly Gly Ala Ala Leu Phe Trp Ser Arg Asp Val Ala
625                 630                 635                 640

Val Val Lys Val Thr Met Asn Phe Glu Pro Asn Lys Val Asn Ile Gln
                645                 650                 655

Lys Lys Asn Cys His Met Glu Gly Lys Glu Thr Val Cys Ile Asn Ala
            660                 665                 670

Thr Val Cys Phe Glu Val Lys Leu Lys Ser Lys Glu Asp Thr Ile Tyr

675                 680                 685

Glu Ala Asp Leu Gln Tyr Arg Val Thr Leu Asp Ser Leu Arg Gln Ile
    690                 695                 700

Ser Arg Ser Phe Phe Ser Gly Thr Gln Glu Arg Lys Val Gln Arg Asn
705                 710                 715                 720

Ile Thr Val Arg Lys Ser Glu Cys Thr Lys His Ser Phe Tyr Met Leu
                725                 730                 735

Asp Lys His Asp Phe Gln Asp Ser Val Arg Ile Thr Leu Asp Phe Asn
            740                 745                 750

Leu Thr Asp Pro Glu Asn Gly Pro Val Leu Asp Asp Ser Leu Pro Asn
        755                 760                 765

Ser Val His Glu Tyr Ile Pro Phe Ala Lys Asp Cys Gly Asn Lys Glu
    770                 775                 780

Lys Cys Ile Ser Asp Leu Ser Leu His Val Ala Thr Thr Glu Lys Asp
785                 790                 795                 800

Leu Leu Ile Val Arg Ser Gln Asn Asp Lys Phe Asn Val Ser Leu Thr
                805                 810                 815

Val Lys Asn Thr Lys Asp Ser Ala Tyr Asn Thr Arg Thr Ile Val His
            820                 825                 830

Tyr Ser Pro Asn Leu Val Phe Ser Gly Ile Glu Ala Ile Gln Lys Asp
        835                 840                 845

Ser Cys Glu Ser Asn His Asn Ile Thr Cys Lys Val Gly Tyr Pro Phe
    850                 855                 860

Leu Arg Arg Gly Glu Met Val Thr Phe Lys Ile Leu Phe Gln Phe Asn
865                 870                 875                 880

Thr Ser Tyr Leu Met Glu Asn Val Thr Ile Tyr Leu Ser Ala Thr Ser
                885                 890                 895

Asp Ser Glu Glu Pro Pro Glu Thr Leu Ser Asp Asn Val Val Asn Ile
            900                 905                 910

Ser Ile Pro Val Lys Tyr Glu Val Gly Leu Gln Phe Tyr Ser Ser Ala
        915                 920                 925

Ser Glu Tyr His Ile Ser Ile Ala Ala Asn Glu Thr Val Pro Glu Val
    930                 935                 940

Ile Asn Ser Thr Glu Asp Ile Gly Asn Glu Ile Asn Ile Phe Tyr Leu

945                 950                 955                 960

Ile Arg Lys Ser Gly Ser Phe Pro Met Pro Glu Leu Lys Leu Ser Ile
                965                 970                 975

Ser Phe Pro Asn Met Thr Ser Asn Gly Tyr Pro Val Leu Tyr Pro Thr
            980                 985                 990

Gly Leu Ser Ser Ser Glu Asn Ala  Asn Cys Arg Pro His  Ile Phe Glu
        995                 1000                 1005

Asp Pro  Phe Ser Ile Asn Ser  Gly Lys Lys Met Thr  Thr Ser Thr
    1010                 1015                 1020

Asp His  Leu Lys Arg Gly Thr  Ile Leu Asp Cys Asn  Thr Cys Lys
    1025                 1030                 1035

Phe Ala  Thr Ile Thr Cys Asn  Leu Thr Ser Ser Asp  Ile Ser Gln
    1040                 1045                 1050

Val Asn  Val Ser Leu Ile Leu  Trp Lys Pro Thr Phe  Ile Lys Ser
    1055                 1060                 1065

Tyr Phe  Ser Ser Leu Asn Leu  Thr Ile Arg Gly Glu  Leu Arg Ser
    1070                 1075                 1080

Glu Asn  Ala Ser Leu Val Leu  Ser Ser Ser Asn Gln  Lys Arg Glu
    1085                 1090                 1095

Leu Ala  Ile Gln Ile Ser Lys  Asp Gly Leu Pro Gly  Arg Val Pro
    1100                 1105                 1110

Leu Trp  Val Ile Leu Leu Ser  Ala Phe Ala Gly Leu  Leu Leu Leu
    1115                 1120                 1125

Met Leu  Leu Ile Leu Ala Leu  Trp Lys Ile Gly Phe  Phe Lys Arg
    1130                 1135                 1140

Pro Leu  Lys Lys Lys Met Glu  Lys
    1145                 1150

<210>  5
<211>  1181
<212>  PRT
<213>  Homo sapiens

<400>  5

Met Gly Pro Glu Arg Thr Gly Ala Ala Pro Leu Pro Leu Leu Leu Val
1               5                   10                  15

Leu Ala Leu Ser Gln Gly Ile Leu Asn Cys Cys Leu Ala Tyr Asn Val
            20                  25                  30

Gly Leu Pro Glu Ala Lys Ile Phe Ser Gly Pro Ser Ser Glu Gln Phe
        35                  40                  45

Gly Tyr Ala Val Gln Gln Phe Ile Asn Pro Lys Gly Asn Trp Leu Leu
    50                  55                  60

Val Gly Ser Pro Trp Ser Gly Phe Pro Glu Asn Arg Met Gly Asp Val
65                  70                  75                  80

Tyr Lys Cys Pro Val Asp Leu Ser Thr Ala Thr Cys Glu Lys Leu Asn
                85                  90                  95

Leu Gln Thr Ser Thr Ser Ile Pro Asn Val Thr Glu Met Lys Thr Asn
            100                 105                 110

Met Ser Leu Gly Leu Ile Leu Thr Arg Asn Met Gly Thr Gly Gly Phe
        115                 120                 125

Leu Thr Cys Gly Pro Leu Trp Ala Gln Gln Cys Gly Asn Gln Tyr Tyr
    130                 135                 140

Thr Thr Gly Val Cys Ser Asp Ile Ser Pro Asp Phe Gln Leu Ser Ala
145                 150                 155                 160

Ser Phe Ser Pro Ala Thr Gln Pro Cys Pro Ser Leu Ile Asp Val Val
                165                 170                 175

Val Val Cys Asp Glu Ser Asn Ser Ile Tyr Pro Trp Asp Ala Val Lys
            180                 185                 190

Asn Phe Leu Glu Lys Phe Val Gln Gly Leu Asp Ile Gly Pro Thr Lys
        195                 200                 205

Thr Gln Val Gly Leu Ile Gln Tyr Ala Asn Asn Pro Arg Val Val Phe
    210                 215                 220

Asn Leu Asn Thr Tyr Lys Thr Lys Glu Glu Met Ile Val Ala Thr Ser
225                 230                 235                 240

Gln Thr Ser Gln Tyr Gly Gly Asp Leu Thr Asn Thr Phe Gly Ala Ile
                245                 250                 255

Gln Tyr Ala Arg Lys Tyr Ala Tyr Ser Ala Ala Ser Gly Gly Arg Arg
            260                 265                 270

Ser Ala Thr Lys Val Met Val Val Val Thr Asp Gly Glu Ser His Asp
        275                 280                 285

Gly Ser Met Leu Lys Ala Val Ile Asp Gln Cys Asn His Asp Asn Ile
    290                 295                 300

Leu Arg Phe Gly Ile Ala Val Leu Gly Tyr Leu Asn Arg Asn Ala Leu
305                 310                 315                 320

Asp Thr Lys Asn Leu Ile Lys Glu Ile Lys Ala Ile Ala Ser Ile Pro
                325                 330                 335

Thr Glu Arg Tyr Phe Phe Asn Val Ser Asp Glu Ala Ala Leu Leu Glu
            340                 345                 350

Lys Ala Gly Thr Leu Gly Glu Gln Ile Phe Ser Ile Glu Gly Thr Val
        355                 360                 365

Gln Gly Gly Asp Asn Phe Gln Met Glu Met Ser Gln Val Gly Phe Ser
    370                 375                 380

Ala Asp Tyr Ser Ser Gln Asn Asp Ile Leu Met Leu Gly Ala Val Gly
385                 390                 395                 400

Ala Phe Gly Trp Ser Gly Thr Ile Val Gln Lys Thr Ser His Gly His
                405                 410                 415

Leu Ile Phe Pro Lys Gln Ala Phe Asp Gln Ile Leu Gln Asp Arg Asn
            420                 425                 430

His Ser Ser Tyr Leu Gly Tyr Ser Val Ala Ala Ile Ser Thr Gly Glu
        435                 440                 445

Ser Thr His Phe Val Ala Gly Ala Pro Arg Ala Asn Tyr Thr Gly Gln
    450                 455                 460

Ile Val Leu Tyr Ser Val Asn Glu Asn Gly Asn Ile Thr Val Ile Gln
465                 470                 475                 480

Ala His Arg Gly Asp Gln Ile Gly Ser Tyr Phe Gly Ser Val Leu Cys
                485                 490                 495

Ser Val Asp Val Asp Lys Asp Thr Ile Thr Asp Val Leu Leu Val Gly
            500                 505                 510

Ala Pro Met Tyr Met Ser Asp Leu Lys Lys Glu Glu Gly Arg Val Tyr
        515                 520                 525

Leu Phe Thr Ile Lys Glu Gly Ile Leu Gly Gln His Gln Phe Leu Glu
    530                 535                 540

Gly Pro Glu Gly Ile Glu Asn Thr Arg Phe Gly Ser Ala Ile Ala Ala
545                 550                 555                 560

Leu Ser Asp Ile Asn Met Asp Gly Phe Asn Asp Val Ile Val Gly Ser
                565                 570                 575

Pro Leu Glu Asn Gln Asn Ser Gly Ala Val Tyr Ile Tyr Asn Gly His
            580                 585                 590

Gln Gly Thr Ile Arg Thr Lys Tyr Ser Gln Lys Ile Leu Gly Ser Asp
        595                 600                 605

Gly Ala Phe Arg Ser His Leu Gln Tyr Phe Gly Arg Ser Leu Asp Gly
    610                 615                 620

Tyr Gly Asp Leu Asn Gly Asp Ser Ile Thr Asp Val Ser Ile Gly Ala
625                 630                 635                 640

Phe Gly Gln Val Val Gln Leu Trp Ser Gln Ser Ile Ala Asp Val Ala
                645                 650                 655

Ile Glu Ala Ser Phe Thr Pro Glu Lys Ile Thr Leu Val Asn Lys Asn
            660                 665                 670

Ala Gln Ile Ile Leu Lys Leu Cys Phe Ser Ala Lys Phe Arg Pro Thr
        675                 680                 685

Lys Gln Asn Asn Gln Val Ala Ile Val Tyr Asn Ile Thr Leu Asp Ala
    690                 695                 700

Asp Gly Phe Ser Ser Arg Val Thr Ser Arg Gly Leu Phe Lys Glu Asn
705                 710                 715                 720

Asn Glu Arg Cys Leu Gln Lys Asn Met Val Val Asn Gln Ala Gln Ser
                725                 730                 735

Cys Pro Glu His Ile Ile Tyr Ile Gln Glu Pro Ser Asp Val Val Asn
            740                 745                 750

Ser Leu Asp Leu Arg Val Asp Ile Ser Leu Glu Asn Pro Gly Thr Ser
        755                 760                 765

Pro Ala Leu Glu Ala Tyr Ser Glu Thr Ala Lys Val Phe Ser Ile Pro
    770                 775                 780

Phe His Lys Asp Cys Gly Glu Asp Gly Leu Cys Ile Ser Asp Leu Val
785                 790                 795                 800

Leu Asp Val Arg Gln Ile Pro Ala Ala Gln Glu Gln Pro Phe Ile Val
                805                 810                 815

Ser Asn Gln Asn Lys Arg Leu Thr Phe Ser Val Thr Leu Lys Asn Lys
            820                 825                 830

Arg Glu Ser Ala Tyr Asn Thr Gly Ile Val Val Asp Phe Ser Glu Asn
        835                 840                 845

Leu Phe Phe Ala Ser Phe Ser Leu Pro Val Asp Gly Thr Glu Val Thr
    850                 855                 860

Cys Gln Val Ala Ala Ser Gln Lys Ser Val Ala Cys Asp Val Gly Tyr
865                 870                 875                 880

Pro Ala Leu Lys Arg Glu Gln Gln Val Thr Phe Thr Ile Asn Phe Asp
                885                 890                 895

Phe Asn Leu Gln Asn Leu Gln Asn Gln Ala Ser Leu Ser Phe Gln Ala
            900                 905                 910

Leu Ser Glu Ser Gln Glu Glu Asn Lys Ala Asp Asn Leu Val Asn Leu
        915                 920                 925

Lys Ile Pro Leu Leu Tyr Asp Ala Glu Ile His Leu Thr Arg Ser Thr
    930                 935                 940

Asn Ile Asn Phe Tyr Glu Ile Ser Ser Asp Gly Asn Val Pro Ser Ile
945                 950                 955                 960

Val His Ser Phe Glu Asp Val Gly Pro Lys Phe Ile Phe Ser Leu Lys
                965                 970                 975

Val Thr Thr Gly Ser Val Pro Val Ser Met Ala Thr Val Ile Ile His
            980                 985                 990

Ile Pro Gln Tyr Thr Lys Glu Lys  Asn Pro Leu Met Tyr  Leu Thr Gly
        995                 1000                 1005

Val Gln  Thr Asp Lys Ala Gly  Asp Ile Ser Cys Asn  Ala Asp Ile
    1010                 1015                 1020

Asn Pro  Leu Lys Ile Gly Gln  Thr Ser Ser Ser Val  Ser Phe Lys
    1025                 1030                 1035

Ser Glu  Asn Phe Arg His Thr  Lys Glu Leu Asn Cys  Arg Thr Ala
    1040                 1045                 1050

Ser Cys  Ser Asn Val Thr Cys  Trp Leu Lys Asp Val  His Met Lys
    1055                 1060                 1065

Gly Glu  Tyr Phe Val Asn Val  Thr Thr Arg Ile Trp  Asn Gly Thr
    1070                 1075                 1080

Phe Ala  Ser Ser Thr Phe Gln  Thr Val Gln Leu Thr  Ala Ala Ala
    1085                 1090                 1095

Glu Ile  Asn Thr Tyr Asn Pro  Glu Ile Tyr Val Ile  Glu Asp Asn
    1100                 1105                 1110

Thr Val Thr Ile Pro Leu Met Ile Met Lys Pro Asp Glu Lys Ala
    1115                1120                1125

Glu Val Pro Thr Gly Val Ile Ile Gly Ser Ile Ile Ala Gly Ile
    1130                1135                1140

Leu Leu Leu Leu Ala Leu Val Ala Ile Leu Trp Lys Leu Gly Phe
    1145                1150                1155

Phe Lys Arg Lys Tyr Glu Lys Met Thr Lys Asn Pro Asp Glu Ile
    1160                1165                1170

Asp Glu Thr Thr Glu Leu Ser Ser
    1175                1180

<210> 6
<211> 1051
<212> PRT
<213> Homo sapiens

<400> 6

<210> 7
<211> 1038
<212> PRT
<213> Homo sapiens

<400> 7

Met Phe Pro Thr Glu Ser Ala Trp Leu Gly Lys Arg Gly Ala Asn Pro
1               5                   10                  15

Gly Pro Glu Ala Ala Val Arg Glu Thr Val Met Leu Leu Leu Cys Leu
            20                  25                  30

Gly Val Pro Thr Gly Arg Pro Tyr Asn Val Asp Thr Glu Ser Ala Leu
        35                  40                  45

Leu Tyr Gln Gly Pro His Asn Thr Leu Phe Gly Tyr Ser Val Val Leu
    50                  55                  60

His Ser His Gly Ala Asn Arg Trp Leu Leu Val Gly Ala Pro Thr Ala
65                  70                  75                  80

Asn Trp Leu Ala Asn Ala Ser Val Ile Asn Pro Gly Ala Ile Tyr Arg
                85                  90                  95

Cys Arg Ile Gly Lys Asn Pro Gly Gln Thr Cys Glu Gln Leu Gln Leu
            100                 105                 110

Gly Ser Pro Asn Gly Glu Pro Cys Gly Lys Thr Cys Leu Glu Glu Arg
        115                 120                 125

Asp Asn Gln Trp Leu Gly Val Thr Leu Ser Arg Gln Pro Gly Glu Asn
    130                 135                 140

Gly Ser Ile Val Thr Cys Gly His Arg Trp Lys Asn Ile Phe Tyr Ile
145                 150                 155                 160

Lys Asn Glu Asn Lys Leu Pro Thr Gly Gly Cys Tyr Gly Val Pro Pro
                165                 170                 175

Asp Leu Arg Thr Glu Leu Ser Lys Arg Ile Ala Pro Cys Tyr Gln Asp
            180                 185                 190

Tyr Val Lys Lys Phe Gly Glu Asn Phe Ala Ser Cys Gln Ala Gly Ile
        195                 200                 205

Ser Ser Phe Tyr Thr Lys Asp Leu Ile Val Met Gly Ala Pro Gly Ser
    210                 215                 220

Ser Tyr Trp Thr Gly Ser Leu Phe Val Tyr Asn Ile Thr Thr Asn Lys
225                 230                 235                 240

Tyr Lys Ala Phe Leu Asp Lys Gln Asn Gln Val Lys Phe Gly Ser Tyr
                245                 250                 255

Leu Gly Tyr Ser Val Gly Ala Gly His Phe Arg Ser Gln His Thr Thr
            260                 265                 270

Glu Val Val Gly Gly Ala Pro Gln His Glu Gln Ile Gly Lys Ala Tyr
        275                 280                 285

Ile Phe Ser Ile Asp Glu Lys Glu Leu Asn Ile Leu His Glu Met Lys
    290                 295                 300

Gly Lys Lys Leu Gly Ser Tyr Phe Gly Ala Ser Val Cys Ala Val Asp
305                 310                 315                 320

Leu Asn Ala Asp Gly Phe Ser Asp Leu Leu Val Gly Ala Pro Met Gln
                325                 330                 335

Ser Thr Ile Arg Glu Glu Gly Arg Val Phe Val Tyr Ile Asn Ser Gly
            340                 345                 350

Ser Gly Ala Val Met Asn Ala Met Glu Thr Asn Leu Val Gly Ser Asp
        355                 360                 365

Lys Tyr Ala Ala Arg Phe Gly Glu Ser Ile Val Asn Leu Gly Asp Ile
    370                 375                 380

Asp Asn Asp Gly Phe Glu Asp Val Ala Ile Gly Ala Pro Gln Glu Asp
385                 390                 395                 400

Asp Leu Gln Gly Ala Ile Tyr Ile Tyr Asn Gly Arg Ala Asp Gly Ile
                405                 410                 415

Ser Ser Thr Phe Ser Gln Arg Ile Glu Gly Leu Gln Ile Ser Lys Ser
            420                 425                 430

Leu Ser Met Phe Gly Gln Ser Ile Ser Gly Gln Ile Asp Ala Asp Asn
        435                 440                 445

Asn Gly Tyr Val Asp Val Ala Val Gly Ala Phe Arg Ser Asp Ser Ala
    450                 455                 460

Val Leu Leu Arg Thr Arg Pro Val Val Ile Val Asp Ala Ser Leu Ser
465                 470                 475                 480

His Pro Glu Ser Val Asn Arg Thr Lys Phe Asp Cys Val Glu Asn Gly
                485                 490                 495

Trp Pro Ser Val Cys Ile Asp Leu Thr Leu Cys Phe Ser Tyr Lys Gly
            500                 505                 510

Lys Glu Val Pro Gly Tyr Ile Val Leu Phe Tyr Asn Met Ser Leu Asp
        515                 520                 525

Val Asn Arg Lys Ala Glu Ser Pro Pro Arg Phe Tyr Phe Ser Ser Asn
    530                 535                 540

Gly Thr Ser Asp Val Ile Thr Gly Ser Ile Gln Val Ser Ser Arg Glu
545                 550                 555                 560

Ala Asn Cys Arg Thr His Gln Ala Phe Met Arg Lys Asp Val Arg Asp
                565                 570                 575

Ile Leu Thr Pro Ile Gln Ile Glu Ala Ala Tyr His Leu Gly Pro His
            580                 585                 590

Val Ile Ser Lys Arg Ser Thr Glu Glu Phe Pro Pro Leu Gln Pro Ile
        595                 600                 605

Leu Gln Gln Lys Lys Glu Lys Asp Ile Met Lys Lys Thr Ile Asn Phe
    610                 615                 620

Ala Arg Phe Cys Ala His Glu Asn Cys Ser Ala Asp Leu Gln Val Ser
625                 630                 635                 640

Ala Lys Ile Gly Phe Leu Lys Pro His Glu Asn Lys Thr Tyr Leu Ala
                645                 650                 655

Val Gly Ser Met Lys Thr Leu Met Leu Asn Val Ser Leu Phe Asn Ala
            660                 665                 670

Gly Asp Asp Ala Tyr Glu Thr Thr Leu His Val Lys Leu Pro Val Gly
        675                 680                 685

Leu Tyr Phe Ile Lys Ile Leu Glu Leu Glu Glu Lys Gln Ile Asn Cys
    690                 695                 700

Glu Val Thr Asp Asn Ser Gly Val Val Gln Leu Asp Cys Ser Ile Gly
705                 710                 715                 720

Tyr Ile Tyr Val Asp His Leu Ser Arg Ile Asp Ile Ser Phe Leu Leu
                725                 730                 735

Asp Val Ser Ser Leu Ser Arg Ala Glu Glu Asp Leu Ser Ile Thr Val
            740                 745                 750

His Ala Thr Cys Glu Asn Glu Glu Glu Met Asp Asn Leu Lys His Ser
        755                 760                 765

Arg Val Thr Val Ala Ile Pro Leu Lys Tyr Glu Val Lys Leu Thr Val
    770                 775                 780

His Gly Phe Val Asn Pro Thr Ser Phe Val Tyr Gly Ser Asn Asp Glu
785                 790                 795                 800

Asn Glu Pro Glu Thr Cys Met Val Glu Lys Met Asn Leu Thr Phe His
                805                 810                 815

Val Ile Asn Thr Gly Asn Ser Met Ala Pro Asn Val Ser Val Glu Ile
            820                 825                 830

Met Val Pro Asn Ser Phe Ser Pro Gln Thr Asp Lys Leu Phe Asn Ile
        835                 840                 845

Leu Asp Val Gln Thr Thr Thr Gly Glu Cys His Phe Glu Asn Tyr Gln
    850                 855                 860

Arg Val Cys Ala Leu Glu Gln Gln Lys Ser Ala Met Gln Thr Leu Lys
865                 870                 875                 880

Gly Ile Val Arg Phe Leu Ser Lys Thr Asp Lys Arg Leu Leu Tyr Cys
                885                 890                 895

Ile Lys Ala Asp Pro His Cys Leu Asn Phe Leu Cys Asn Phe Gly Lys
            900                 905                 910

Met Glu Ser Gly Lys Glu Ala Ser Val His Ile Gln Leu Glu Gly Arg
        915                 920                 925

Pro Ser Ile Leu Glu Met Asp Glu Thr Ser Ala Leu Lys Phe Glu Ile
    930                 935                 940

Arg Ala Thr Gly Phe Pro Glu Pro Asn Pro Arg Val Ile Glu Leu Asn
945                 950                 955                 960

Lys Asp Glu Asn Val Ala His Val Leu Leu Glu Gly Leu His His Gln
                965                 970                 975

Arg Pro Lys Arg Tyr Phe Thr Ile Val Ile Ile Ser Ser Ser Leu Leu
            980                 985                 990

Leu Gly Leu Ile Val Leu Leu Leu Ile Ser Tyr Val Met Trp Lys Ala
        995                 1000                1005

Gly Phe Phe Lys Arg Gln Tyr Lys Ser Ile Leu Gln Glu Glu Asn
    1010                1015                1020

Arg Arg Asp Ser Trp Ser Tyr Ile Asn Ser Lys Ser Asn Asp Asp
    1025                1030                1035

<210> 8
<211> 1049
<212> PRT
<213> Homo sapiens

<400> 8

Met Gly Ser Arg Thr Pro Glu Ser Pro Leu His Ala Val Gln Leu Arg
1               5                   10                  15

Trp Gly Pro Arg Arg Arg Pro Pro Leu Leu Pro Leu Leu Leu Leu Leu
            20                  25                  30

Leu Pro Pro Pro Pro Arg Val Gly Gly Phe Asn Leu Asp Ala Glu Ala
        35                  40                  45

Pro Ala Val Leu Ser Gly Pro Pro Gly Ser Phe Phe Gly Phe Ser Val
    50                  55                  60

Glu Phe Tyr Arg Pro Gly Thr Asp Gly Val Ser Val Leu Val Gly Ala
65                  70                  75                  80

Pro Lys Ala Asn Thr Ser Gln Pro Gly Val Leu Gln Gly Gly Ala Val
                85                  90                  95

Tyr Leu Cys Pro Trp Gly Ala Ser Pro Thr Gln Cys Thr Pro Ile Glu
            100                 105                 110

Phe Asp Ser Lys Gly Ser Arg Leu Leu Glu Ser Ser Leu Ser Ser Ser
        115                 120                 125

Glu Gly Glu Glu Pro Val Glu Tyr Lys Ser Leu Gln Trp Phe Gly Ala
    130                 135                 140

Thr Val Arg Ala His Gly Ser Ser Ile Leu Ala Cys Ala Pro Leu Tyr
145                 150                 155                 160

Ser Trp Arg Thr Glu Lys Glu Pro Leu Ser Asp Pro Val Gly Thr Cys
                165                 170                 175

Tyr Leu Ser Thr Asp Asn Phe Thr Arg Ile Leu Glu Tyr Ala Pro Cys

180                 185                 190

Arg Ser Asp Phe Ser Trp Ala Ala Gly Gln Gly Tyr Cys Gln Gly Gly
        195                 200                 205

Phe Ser Ala Glu Phe Thr Lys Thr Gly Arg Val Val Leu Gly Gly Pro
    210                 215                 220

Gly Ser Tyr Phe Trp Gln Gly Gln Ile Leu Ser Ala Thr Gln Glu Gln
225                 230                 235                 240

Ile Ala Glu Ser Tyr Tyr Pro Glu Tyr Leu Ile Asn Leu Val Gln Gly
                245                 250                 255

Gln Leu Gln Thr Arg Gln Ala Ser Ser Ile Tyr Asp Asp Ser Tyr Leu
            260                 265                 270

Gly Tyr Ser Val Ala Val Gly Glu Phe Ser Gly Asp Asp Thr Glu Asp
        275                 280                 285

Phe Val Ala Gly Val Pro Lys Gly Asn Leu Thr Tyr Gly Tyr Val Thr
    290                 295                 300

Ile Leu Asn Gly Ser Asp Ile Arg Ser Leu Tyr Asn Phe Ser Gly Glu
305                 310                 315                 320

Gln Met Ala Ser Tyr Phe Gly Tyr Ala Val Ala Ala Thr Asp Val Asn
                325                 330                 335

Gly Asp Gly Leu Asp Asp Leu Leu Val Gly Ala Pro Leu Leu Met Asp
            340                 345                 350

Arg Thr Pro Asp Gly Arg Pro Gln Glu Val Gly Arg Val Tyr Val Tyr
        355                 360                 365

Leu Gln His Pro Ala Gly Ile Glu Pro Thr Pro Thr Leu Thr Leu Thr
    370                 375                 380

Gly His Asp Glu Phe Gly Arg Phe Gly Ser Ser Leu Thr Pro Leu Gly
385                 390                 395                 400

Asp Leu Asp Gln Asp Gly Tyr Asn Asp Val Ala Ile Gly Ala Pro Phe
                405                 410                 415

Gly Gly Glu Thr Gln Gln Gly Val Val Phe Val Phe Pro Gly Gly Pro
            420                 425                 430

Gly Gly Leu Gly Ser Lys Pro Ser Gln Val Leu Gln Pro Leu Trp Ala
        435                 440                 445

Ala Ser His Thr Pro Asp Phe Phe Gly Ser Ala Leu Arg Gly Gly Arg

    450                 455                 460

Asp Leu Asp Gly Asn Gly Tyr Pro Asp Leu Ile Val Gly Ser Phe Gly
465                 470                 475                 480

Val Asp Lys Ala Val Val Tyr Arg Gly Arg Pro Ile Val Ser Ala Ser
                485                 490                 495

Ala Ser Leu Thr Ile Phe Pro Ala Met Phe Asn Pro Glu Glu Arg Ser
            500                 505                 510

Cys Ser Leu Glu Gly Asn Pro Val Ala Cys Ile Asn Leu Ser Phe Cys
        515                 520                 525

Leu Asn Ala Ser Gly Lys His Val Ala Asp Ser Ile Gly Phe Thr Val
    530                 535                 540

Glu Leu Gln Leu Asp Trp Gln Lys Gln Lys Gly Gly Val Arg Arg Ala
545                 550                 555                 560

Leu Phe Leu Ala Ser Arg Gln Ala Thr Leu Thr Gln Thr Leu Leu Ile
                565                 570                 575

Gln Asn Gly Ala Arg Glu Asp Cys Arg Glu Met Lys Ile Tyr Leu Arg
            580                 585                 590

Asn Glu Ser Glu Phe Arg Asp Lys Leu Ser Pro Ile His Ile Ala Leu
        595                 600                 605

Asn Phe Ser Leu Asp Pro Gln Ala Pro Val Asp Ser His Gly Leu Arg
    610                 615                 620

Pro Ala Leu His Tyr Gln Ser Lys Ser Arg Ile Glu Asp Lys Ala Gln
625                 630                 635                 640

Ile Leu Leu Asp Cys Gly Glu Asp Asn Ile Cys Val Pro Asp Leu Gln
                645                 650                 655

Leu Glu Val Phe Gly Glu Gln Asn His Val Tyr Leu Gly Asp Lys Asn
            660                 665                 670

Ala Leu Asn Leu Thr Phe His Ala Gln Asn Val Gly Glu Gly Gly Ala
        675                 680                 685

Tyr Glu Ala Glu Leu Arg Val Thr Ala Pro Pro Glu Ala Glu Tyr Ser
    690                 695                 700

Gly Leu Val Arg His Pro Gly Asn Phe Ser Ser Leu Ser Cys Asp Tyr
705                 710                 715                 720

Phe Ala Val Asn Gln Ser Arg Leu Leu Val Cys Asp Leu Gly Asn Pro

                725                 730                 735

Met Lys Ala Gly Ala Ser Leu Trp Gly Gly Leu Arg Phe Thr Val Pro
            740                 745                 750

His Leu Arg Asp Thr Lys Lys Thr Ile Gln Phe Asp Phe Gln Ile Leu
        755                 760                 765

Ser Lys Asn Leu Asn Asn Ser Gln Ser Asp Val Val Ser Phe Arg Leu
    770                 775                 780

Ser Val Glu Ala Gln Ala Gln Val Thr Leu Asn Gly Val Ser Lys Pro
785                 790                 795                 800

Glu Ala Val Leu Phe Pro Val Ser Asp Trp His Pro Arg Asp Gln Pro
                805                 810                 815

Gln Lys Glu Glu Asp Leu Gly Pro Ala Val His His Val Tyr Glu Leu
            820                 825                 830

Ile Asn Gln Gly Pro Ser Ser Ile Ser Gln Gly Val Leu Glu Leu Ser
        835                 840                 845

Cys Pro Gln Ala Leu Glu Gly Gln Gln Leu Leu Tyr Val Thr Arg Val
    850                 855                 860

Thr Gly Leu Asn Cys Thr Thr Asn His Pro Ile Asn Pro Lys Gly Leu
865                 870                 875                 880

Glu Leu Asp Pro Glu Gly Ser Leu His His Gln Gln Lys Arg Glu Ala
                885                 890                 895

Pro Ser Arg Ser Ser Ala Ser Ser Gly Pro Gln Ile Leu Lys Cys Pro
            900                 905                 910

Glu Ala Glu Cys Phe Arg Leu Arg Cys Glu Leu Gly Pro Leu His Gln
        915                 920                 925

Gln Glu Ser Gln Ser Leu Gln Leu His Phe Arg Val Trp Ala Lys Thr
    930                 935                 940

Phe Leu Gln Arg Glu His Gln Pro Phe Ser Leu Gln Cys Glu Ala Val
945                 950                 955                 960

Tyr Lys Ala Leu Lys Met Pro Tyr Arg Ile Leu Pro Arg Gln Leu Pro
                965                 970                 975

Gln Lys Glu Arg Gln Val Ala Thr Ala Val Gln Trp Thr Lys Ala Glu
            980                 985                 990

Gly Ser Tyr Gly Val Pro Leu Trp Ile Ile Ile Leu Ala Ile Leu Phe

995                 1000                1005

Gly Leu Leu Leu Leu Gly Leu Leu Ile Tyr Ile Leu Tyr Lys Leu
    1010                1015                1020

Gly Phe Phe Lys Arg Ser Leu Pro Tyr Gly Thr Ala Met Glu Lys
    1025                1030                1035

Ala Gln Leu Lys Pro Pro Ala Thr Ser Asp Ala
    1040                1045

<210>  9
<211>  1073
<212>  PRT
<213>  Homo sapiens

<400>  9

Met Ala Ala Ala Gly Gln Leu Cys Leu Leu Tyr Leu Ser Ala Gly Leu
1               5                   10                  15

Leu Ser Arg Leu Gly Ala Ala Phe Asn Leu Asp Thr Arg Glu Asp Asn
            20                  25                  30

Val Ile Arg Lys Tyr Gly Asp Pro Gly Ser Leu Phe Gly Phe Ser Leu
        35                  40                  45

Ala Met His Trp Gln Leu Gln Pro Glu Asp Lys Arg Leu Leu Leu Val
    50                  55                  60

Gly Ala Pro Arg Gly Glu Ala Leu Pro Leu Gln Arg Ala Asn Arg Thr
65                  70                  75                  80

Gly Gly Leu Tyr Ser Cys Asp Ile Thr Ala Arg Gly Pro Cys Thr Arg
                85                  90                  95

Ile Glu Phe Asp Asn Asp Ala Asp Pro Thr Ser Glu Ser Lys Glu Asp
            100                 105                 110

Gln Trp Met Gly Val Thr Val Gln Ser Gln Gly Pro Gly Gly Lys Val
        115                 120                 125

Val Thr Cys Ala His Arg Tyr Glu Lys Arg Gln His Val Asn Thr Lys
    130                 135                 140

Gln Glu Ser Arg Asp Ile Phe Gly Arg Cys Tyr Val Leu Ser Gln Asn
145                 150                 155                 160

Leu Arg Ile Glu Asp Asp Met Asp Gly Gly Asp Trp Ser Phe Cys Asp
                165                 170                 175

Gly Arg Leu Arg Gly His Glu Lys Phe Gly Ser Cys Gln Gln Gly Val
            180                 185                 190

Ala Ala Thr Phe Thr Lys Asp Phe His Tyr Ile Val Phe Gly Ala Pro
        195                 200                 205

Gly Thr Tyr Asn Trp Lys Gly Ile Val Arg Val Glu Gln Lys Asn Asn
    210                 215                 220

Thr Phe Phe Asp Met Asn Ile Phe Glu Asp Gly Pro Tyr Glu Val Gly
225                 230                 235                 240

Gly Glu Thr Glu His Asp Glu Ser Leu Val Pro Val Pro Ala Asn Ser
                245                 250                 255

Tyr Leu Gly Phe Ser Leu Asp Ser Gly Lys Gly Ile Val Ser Lys Asp
            260                 265                 270

Glu Ile Thr Phe Val Ser Gly Ala Pro Arg Ala Asn His Ser Gly Ala
        275                 280                 285

Val Val Leu Leu Lys Arg Asp Met Lys Ser Ala His Leu Leu Pro Glu
    290                 295                 300

His Ile Phe Asp Gly Glu Gly Leu Ala Ser Ser Phe Gly Tyr Asp Val
305                 310                 315                 320

Ala Val Val Asp Leu Asn Lys Asp Gly Trp Gln Asp Ile Val Ile Gly
                325                 330                 335

Ala Pro Gln Tyr Phe Asp Arg Asp Gly Glu Val Gly Gly Ala Val Tyr
            340                 345                 350

Val Tyr Met Asn Gln Gln Gly Arg Trp Asn Asn Val Lys Pro Ile Arg
        355                 360                 365

Leu Asn Gly Thr Lys Asp Ser Met Phe Gly Ile Ala Val Lys Asn Ile
    370                 375                 380

Gly Asp Ile Asn Gln Asp Gly Tyr Pro Asp Ile Ala Val Gly Ala Pro
385                 390                 395                 400

Tyr Asp Asp Leu Gly Lys Val Phe Ile Tyr His Gly Ser Ala Asn Gly
                405                 410                 415

Ile Asn Thr Lys Pro Thr Gln Val Leu Lys Gly Ile Ser Pro Tyr Phe
            420                 425                 430

Gly Tyr Ser Ile Ala Gly Asn Met Asp Leu Asp Arg Asn Ser Tyr Pro
        435                 440                 445

Asp Val Ala Val Gly Ser Leu Ser Asp Ser Val Thr Ile Phe Arg Ser
    450                 455                 460

Arg Pro Val Ile Asn Ile Gln Lys Thr Ile Thr Val Thr Pro Asn Arg
465                 470                 475                 480

Ile Asp Leu Arg Gln Lys Thr Ala Cys Gly Ala Pro Ser Gly Ile Cys
                485                 490                 495

Leu Gln Val Lys Ser Cys Phe Glu Tyr Thr Ala Asn Pro Ala Gly Tyr
            500                 505                 510

Asn Pro Ser Ile Ser Ile Val Gly Thr Leu Glu Ala Glu Lys Glu Arg
        515                 520                 525

Arg Lys Ser Gly Leu Ser Ser Arg Val Gln Phe Arg Asn Gln Gly Ser
    530                 535                 540

Glu Pro Lys Tyr Thr Gln Glu Leu Thr Leu Lys Arg Gln Lys Gln Lys
545                 550                 555                 560

Val Cys Met Glu Glu Thr Leu Trp Leu Gln Asp Asn Ile Arg Asp Lys
                565                 570                 575

Leu Arg Pro Ile Pro Ile Thr Ala Ser Val Glu Ile Gln Glu Pro Ser
            580                 585                 590

Ser Arg Arg Arg Val Asn Ser Leu Pro Glu Val Leu Pro Ile Leu Asn
        595                 600                 605

Ser Asp Glu Pro Lys Thr Ala His Ile Asp Val His Phe Leu Lys Glu
    610                 615                 620

Gly Cys Gly Asp Asp Asn Val Cys Asn Ser Asn Leu Lys Leu Glu Tyr
625                 630                 635                 640

Lys Phe Cys Thr Arg Glu Gly Asn Gln Asp Lys Phe Ser Tyr Leu Pro
                645                 650                 655

Ile Gln Lys Gly Val Pro Glu Leu Val Leu Lys Asp Gln Lys Asp Ile
            660                 665                 670

Ala Leu Glu Ile Thr Val Thr Asn Ser Pro Ser Asn Pro Arg Asn Pro
        675                 680                 685

Thr Lys Asp Gly Asp Asp Ala His Glu Ala Lys Leu Ile Ala Thr Phe
    690                 695                 700

Pro Asp Thr Leu Thr Tyr Ser Ala Tyr Arg Glu Leu Arg Ala Phe Pro
705                 710                 715                 720

Glu Lys Gln Leu Ser Cys Val Ala Asn Gln Asn Gly Ser Gln Ala Asp
                725                 730                 735

Cys Glu Leu Gly Asn Pro Phe Lys Arg Asn Ser Asn Val Thr Phe Tyr
            740                 745                 750

Leu Val Leu Ser Thr Thr Glu Val Thr Phe Asp Thr Pro Tyr Leu Asp
        755                 760                 765

Ile Asn Leu Lys Leu Glu Thr Thr Ser Asn Gln Asp Asn Leu Ala Pro
    770                 775                 780

Ile Thr Ala Lys Ala Lys Val Val Ile Glu Leu Leu Leu Ser Val Ser
785                 790                 795                 800

Gly Val Ala Lys Pro Ser Gln Val Tyr Phe Gly Gly Thr Val Val Gly
                805                 810                 815

Glu Gln Ala Met Lys Ser Glu Asp Glu Val Gly Ser Leu Ile Glu Tyr
            820                 825                 830

Glu Phe Arg Val Ile Asn Leu Gly Lys Pro Leu Thr Asn Leu Gly Thr
        835                 840                 845

Ala Thr Leu Asn Ile Gln Trp Pro Lys Glu Ile Ser Asn Gly Lys Trp
    850                 855                 860

Leu Leu Tyr Leu Val Lys Val Glu Ser Lys Gly Leu Glu Lys Val Thr
865                 870                 875                 880

Cys Glu Pro Gln Lys Glu Ile Asn Ser Leu Asn Leu Thr Glu Ser His
                885                 890                 895

Asn Ser Arg Lys Lys Arg Glu Ile Thr Glu Lys Gln Ile Asp Asp Asn
            900                 905                 910

Arg Lys Phe Ser Leu Phe Ala Glu Arg Lys Tyr Gln Thr Leu Asn Cys
        915                 920                 925

Ser Val Asn Val Asn Cys Val Asn Ile Arg Cys Pro Leu Arg Gly Leu
    930                 935                 940

Asp Ser Lys Ala Ser Leu Ile Leu Arg Ser Arg Leu Trp Asn Ser Thr
945                 950                 955                 960

Phe Leu Glu Glu Tyr Ser Lys Leu Asn Tyr Leu Asp Ile Leu Met Arg
                965                 970                 975

Ala Phe Ile Asp Val Thr Ala Ala Ala Glu Asn Ile Arg Leu Pro Asn
            980                 985                 990

Ala Gly Thr Gln Val Arg Val Thr  Val Phe Pro Ser Lys  Thr Val Ala
        995                 1000                1005

Gln Tyr Ser Gly Val Pro Trp Trp Ile Ile Leu Val Ala Ile Leu
    1010                1015                1020

Ala Gly Ile Leu Met Leu Ala Leu Leu Val Phe Ile Leu Trp Lys
    1025                1030                1035

Cys Gly Phe Phe Lys Arg Asn Lys Lys Asp His Tyr Asp Ala Thr
    1040                1045                1050

Tyr His Lys Ala Glu Ile His Ala Gln Pro Ser Asp Lys Glu Arg
    1055                1060                1065

Leu Thr Ser Asp Ala
    1070

<210>  10
<211>  789
<212>  PRT
<213>  Homo sapiens

<400>  10

Met Asn Leu Gln Pro Ile Phe Trp Ile Gly Leu Ile Ser Ser Val Cys
1               5                   10                  15

Cys Val Phe Ala Gln Thr Asp Glu Asn Arg Cys Leu Lys Ala Asn Ala
            20                  25                  30

Lys Ser Cys Gly Glu Cys Ile Gln Ala Gly Pro Asn Cys Gly Trp Cys
        35                  40                  45

Thr Asn Ser Thr Phe Leu Gln Glu Gly Met Pro Thr Ser Ala Arg Cys
    50                  55                  60

Asp Asp Leu Glu Ala Leu Lys Lys Lys Gly Cys Pro Pro Asp Asp Ile
65                  70                  75                  80

Glu Asn Pro Arg Gly Ser Lys Asp Ile Lys Lys Asn Lys Asn Val Thr
                85                  90                  95

Asn Arg Ser Lys Gly Thr Ala Glu Lys Leu Lys Pro Glu Asp Ile Thr
            100                 105                 110

Gln Ile Gln Pro Gln Gln Leu Val Leu Arg Leu Arg Ser Gly Glu Pro
        115                 120                 125

Gln Thr Phe Thr Leu Lys Phe Lys Arg Ala Glu Asp Tyr Pro Ile Asp
    130                 135                 140

Leu Tyr Tyr Leu Met Asp Leu Ser Tyr Ser Met Lys Asp Asp Leu Glu
145                 150                 155                 160

Asn Val Lys Ser Leu Gly Thr Asp Leu Met Asn Glu Met Arg Arg Ile
                165                 170                 175

Thr Ser Asp Phe Arg Ile Gly Phe Gly Ser Phe Val Glu Lys Thr Val
            180                 185                 190

Met Pro Tyr Ile Ser Thr Thr Pro Ala Lys Leu Arg Asn Pro Cys Thr
        195                 200                 205

Ser Glu Gln Asn Cys Thr Ser Pro Phe Ser Tyr Lys Asn Val Leu Ser
    210                 215                 220

Leu Thr Asn Lys Gly Glu Val Phe Asn Glu Leu Val Gly Lys Gln Arg
225                 230                 235                 240

Ile Ser Gly Asn Leu Asp Ser Pro Glu Gly Gly Phe Asp Ala Ile Met
                245                 250                 255

Gln Val Ala Val Cys Gly Ser Leu Ile Gly Trp Arg Asn Val Thr Arg
            260                 265                 270

Leu Leu Val Phe Ser Thr Asp Ala Gly Phe His Phe Ala Gly Asp Gly
        275                 280                 285

Lys Leu Gly Gly Ile Val Leu Pro Asn Asp Gly Gln Cys His Leu Glu
    290                 295                 300

Asn Asn Met Tyr Thr Met Ser His Tyr Tyr Asp Tyr Pro Ser Ile Ala
305                 310                 315                 320

His Leu Val Gln Lys Leu Ser Glu Asn Asn Ile Gln Thr Ile Phe Ala
                325                 330                 335

Val Thr Glu Glu Phe Gln Pro Val Tyr Lys Glu Leu Lys Asn Leu Ile
            340                 345                 350

Pro Lys Ser Ala Val Gly Thr Leu Ser Ala Asn Ser Ser Asn Val Ile
        355                 360                 365

Gln Leu Ile Ile Asp Ala Tyr Asn Ser Leu Ser Ser Glu Val Ile Leu
    370                 375                 380

Glu Asn Gly Lys Leu Ser Glu Gly Val Thr Ile Ser Tyr Lys Ser Tyr
385                 390                 395                 400

Cys Lys Asn Gly Val Asn Gly Thr Gly Glu Asn Gly Arg Lys Cys Ser
                405                 410                 415

Asn Ile Ser Ile Gly Asp Glu Val Gln Phe Glu Ile Ser Ile Thr Ser
            420                 425                 430

Asn Lys Cys Pro Lys Lys Asp Ser Asp Ser Phe Lys Ile Arg Pro Leu
        435                 440                 445

Gly Phe Thr Glu Glu Val Glu Val Ile Leu Gln Tyr Ile Cys Glu Cys
    450                 455                 460

Glu Cys Gln Ser Glu Gly Ile Pro Glu Ser Pro Lys Cys His Glu Gly
465                 470                 475                 480

Asn Gly Thr Phe Glu Cys Gly Ala Cys Arg Cys Asn Glu Gly Arg Val
                485                 490                 495

Gly Arg His Cys Glu Cys Ser Thr Asp Glu Val Asn Ser Glu Asp Met
            500                 505                 510

Asp Ala Tyr Cys Arg Lys Glu Asn Ser Ser Glu Ile Cys Ser Asn Asn
        515                 520                 525

Gly Glu Cys Val Cys Gly Gln Cys Val Cys Arg Lys Arg Asp Asn Thr
    530                 535                 540

Asn Glu Ile Tyr Ser Gly Lys Phe Cys Glu Cys Asp Asn Phe Asn Cys
545                 550                 555                 560

Asp Arg Ser Asn Gly Leu Ile Cys Gly Gly Asn Gly Val Cys Lys Cys
                565                 570                 575

Arg Val Cys Glu Cys Asn Pro Asn Tyr Thr Gly Ser Ala Cys Asp Cys
            580                 585                 590

Ser Leu Asp Thr Ser Thr Cys Glu Ala Ser Asn Gly Gln Ile Cys Asn
        595                 600                 605

Gly Arg Gly Ile Cys Glu Cys Gly Val Cys Lys Cys Thr Asp Pro Lys
    610                 615                 620

Phe Gln Gly Gln Thr Cys Glu Met Cys Gln Thr Cys Leu Gly Val Cys
625                 630                 635                 640

Ala Glu His Lys Glu Cys Val Gln Cys Arg Ala Phe Asn Lys Gly Glu
                645                 650                 655

Lys Lys Asp Thr Cys Thr Gln Glu Cys Ser Tyr Phe Asn Ile Thr Lys
            660                 665                 670

Val Glu Ser Arg Asp Lys Leu Pro Gln Pro Val Gln Pro Asp Pro Val
        675                 680                 685

Ser His Cys Lys Glu Lys Asp Val Asp Asp Cys Trp Phe Tyr Phe Thr
    690                 695                 700

Tyr Ser Val Asn Gly Asn Asn Glu Val Met Val His Val Val Glu Asn
705                 710                 715                 720

Pro Glu Cys Pro Thr Gly Pro Asp Ile Ile Pro Ile Val Ala Gly Val
                725                 730                 735

Val Ala Gly Ile Val Leu Ile Gly Leu Ala Leu Leu Leu Ile Trp Lys
            740                 745                 750

Leu Leu Met Ile Ile His Asp Arg Arg Glu Phe Ala Lys Phe Glu Lys
        755                 760                 765

Glu Lys Met Asn Ala Lys Trp Asp Thr Val Ser Tyr Lys Thr Ser Lys
    770                 775                 780

Lys Gln Ser Gly Leu
785

<210>  11
<211>  825
<212>  PRT
<213>  Homo sapiens

<400>  11

Met Asn Leu Gln Pro Ile Phe Trp Ile Gly Leu Ile Ser Ser Val Cys
1               5                   10                  15

Cys Val Phe Ala Gln Thr Asp Glu Asn Arg Cys Leu Lys Ala Asn Ala
            20                  25                  30

Lys Ser Cys Gly Glu Cys Ile Gln Ala Gly Pro Asn Cys Gly Trp Cys
        35                  40                  45

Thr Asn Ser Thr Phe Leu Gln Glu Gly Met Pro Thr Ser Ala Arg Cys
    50                  55                  60

Asp Asp Leu Glu Ala Leu Lys Lys Lys Gly Cys Pro Pro Asp Asp Ile
65                  70                  75                  80

Glu Asn Pro Arg Gly Ser Lys Asp Ile Lys Lys Asn Lys Asn Val Thr
                85                  90                  95

Asn Arg Ser Lys Gly Thr Ala Glu Lys Leu Lys Pro Glu Asp Ile Thr
            100                 105                 110

Gln Ile Gln Pro Gln Gln Leu Val Leu Arg Leu Arg Ser Gly Glu Pro
        115                 120                 125

Gln Thr Phe Thr Leu Lys Phe Lys Arg Ala Glu Asp Tyr Pro Ile Asp
    130                 135                 140

Leu Tyr Tyr Leu Met Asp Leu Ser Tyr Ser Met Lys Asp Asp Leu Glu
145                 150                 155                 160

Asn Val Lys Ser Leu Gly Thr Asp Leu Met Asn Glu Met Arg Arg Ile
                165                 170                 175

Thr Ser Asp Phe Arg Ile Gly Phe Gly Ser Phe Val Glu Lys Thr Val
            180                 185                 190

Met Pro Tyr Ile Ser Thr Thr Pro Ala Lys Leu Arg Asn Pro Cys Thr
        195                 200                 205

Ser Glu Gln Asn Cys Thr Ser Pro Phe Ser Tyr Lys Asn Val Leu Ser
    210                 215                 220

Leu Thr Asn Lys Gly Glu Val Phe Asn Glu Leu Val Gly Lys Gln Arg
225                 230                 235                 240

Ile Ser Gly Asn Leu Asp Ser Pro Glu Gly Gly Phe Asp Ala Ile Met
                245                 250                 255

Gln Val Ala Val Cys Gly Ser Leu Ile Gly Trp Arg Asn Val Thr Arg
            260                 265                 270

Leu Leu Val Phe Ser Thr Asp Ala Gly Phe His Phe Ala Gly Asp Gly
        275                 280                 285

Lys Leu Gly Gly Ile Val Leu Pro Asn Asp Gly Gln Cys His Leu Glu
    290                 295                 300

Asn Asn Met Tyr Thr Met Ser His Tyr Tyr Asp Tyr Pro Ser Ile Ala
305                 310                 315                 320

His Leu Val Gln Lys Leu Ser Glu Asn Asn Ile Gln Thr Ile Phe Ala
                325                 330                 335

Val Thr Glu Glu Phe Gln Pro Val Tyr Lys Glu Leu Lys Asn Leu Ile
            340                 345                 350

Pro Lys Ser Ala Val Gly Thr Leu Ser Ala Asn Ser Ser Asn Val Ile
        355                 360                 365

Gln Leu Ile Ile Asp Ala Tyr Asn Ser Leu Ser Ser Glu Val Ile Leu
    370                 375                 380

Glu Asn Gly Lys Leu Ser Glu Gly Val Thr Ile Ser Tyr Lys Ser Tyr
385                 390                 395                 400

Cys Lys Asn Gly Val Asn Gly Thr Gly Glu Asn Gly Arg Lys Cys Ser
                405                 410                 415

Asn Ile Ser Ile Gly Asp Glu Val Gln Phe Glu Ile Ser Ile Thr Ser
            420                 425                 430

Asn Lys Cys Pro Lys Lys Asp Ser Asp Ser Phe Lys Ile Arg Pro Leu
        435                 440                 445

Gly Phe Thr Glu Glu Val Glu Val Ile Leu Gln Tyr Ile Cys Glu Cys
    450                 455                 460

Glu Cys Gln Ser Glu Gly Ile Pro Glu Ser Pro Lys Cys His Glu Gly
465                 470                 475                 480

Asn Gly Thr Phe Glu Cys Gly Ala Cys Arg Cys Asn Glu Gly Arg Val
                485                 490                 495

Gly Arg His Cys Glu Cys Ser Thr Asp Glu Val Asn Ser Glu Asp Met
            500                 505                 510

Asp Ala Tyr Cys Arg Lys Glu Asn Ser Ser Glu Ile Cys Ser Asn Asn
        515                 520                 525

Gly Glu Cys Val Cys Gly Gln Cys Val Cys Arg Lys Arg Asp Asn Thr
    530                 535                 540

Asn Glu Ile Tyr Ser Gly Lys Phe Cys Glu Cys Asp Asn Phe Asn Cys
545                 550                 555                 560

Asp Arg Ser Asn Gly Leu Ile Cys Gly Gly Asn Gly Val Cys Lys Cys
                565                 570                 575

Arg Val Cys Glu Cys Asn Pro Asn Tyr Thr Gly Ser Ala Cys Asp Cys
            580                 585                 590

Ser Leu Asp Thr Ser Thr Cys Glu Ala Ser Asn Gly Gln Ile Cys Asn
        595                 600                 605

Gly Arg Gly Ile Cys Glu Cys Gly Val Cys Lys Cys Thr Asp Pro Lys
    610                 615                 620

Phe Gln Gly Gln Thr Cys Glu Met Cys Gln Thr Cys Leu Gly Val Cys
625                 630                 635                 640

Ala Glu His Lys Glu Cys Val Gln Cys Arg Ala Phe Asn Lys Gly Glu
                645                 650                 655

Lys Lys Asp Thr Cys Thr Gln Glu Cys Ser Tyr Phe Asn Ile Thr Lys
            660                 665                 670

Val Glu Ser Arg Asp Lys Leu Pro Gln Pro Val Gln Pro Asp Pro Val
        675                 680                 685

Ser His Cys Lys Glu Lys Asp Val Asp Asp Cys Trp Phe Tyr Phe Thr
    690                 695                 700

Tyr Ser Val Asn Gly Asn Asn Glu Val Met Val His Val Val Glu Asn
705                 710                 715                 720

Pro Glu Cys Pro Thr Gly Pro Asp Ile Ile Pro Ile Val Ala Gly Val
                725                 730                 735

Val Ala Gly Ile Val Leu Ile Gly Leu Ala Leu Leu Leu Ile Trp Lys
            740                 745                 750

Leu Leu Met Ile Ile His Asp Arg Arg Glu Phe Ala Lys Phe Glu Lys
        755                 760                 765

Glu Lys Met Asn Ala Lys Trp Asp Thr Ser Leu Ser Val Ala Gln Pro
    770                 775                 780

Gly Val Gln Trp Cys Asp Ile Ser Ser Leu Gln Pro Leu Thr Ser Arg
785                 790                 795                 800

Phe Gln Gln Phe Ser Cys Leu Ser Leu Pro Ser Thr Trp Asp Tyr Arg
                805                 810                 815

Val Lys Ile Leu Phe Ile Arg Val Pro
            820                 825

<210>  12
<211>  819
<212>  PRT
<213>  Homo sapiens

<400>  12

Met Asn Leu Gln Pro Ile Phe Trp Ile Gly Leu Ile Ser Ser Val Cys
1               5                   10                  15

Cys Val Phe Ala Gln Thr Asp Glu Asn Arg Cys Leu Lys Ala Asn Ala
            20                  25                  30

Lys Ser Cys Gly Glu Cys Ile Gln Ala Gly Pro Asn Cys Gly Trp Cys
        35                  40                  45

Thr Asn Ser Thr Phe Leu Gln Glu Gly Met Pro Thr Ser Ala Arg Cys
    50                  55                  60

Asp Asp Leu Glu Ala Leu Lys Lys Lys Gly Cys Pro Pro Asp Asp Ile
65                  70                  75                  80

Glu Asn Pro Arg Gly Ser Lys Asp Ile Lys Lys Asn Lys Asn Val Thr
                85                  90                  95

Asn Arg Ser Lys Gly Thr Ala Glu Lys Leu Lys Pro Glu Asp Ile Thr

            100                 105                 110

Gln Ile Gln Pro Gln Gln Leu Val Leu Arg Leu Arg Ser Gly Glu Pro
        115                 120                 125

Gln Thr Phe Thr Leu Lys Phe Lys Arg Ala Glu Asp Tyr Pro Ile Asp
    130                 135                 140

Leu Tyr Tyr Leu Met Asp Leu Ser Tyr Ser Met Lys Asp Asp Leu Glu
145                 150                 155                 160

Asn Val Lys Ser Leu Gly Thr Asp Leu Met Asn Glu Met Arg Arg Ile
                165                 170                 175

Thr Ser Asp Phe Arg Ile Gly Phe Gly Ser Phe Val Glu Lys Thr Val
            180                 185                 190

Met Pro Tyr Ile Ser Thr Thr Pro Ala Lys Leu Arg Asn Pro Cys Thr
        195                 200                 205

Ser Glu Gln Asn Cys Thr Ser Pro Phe Ser Tyr Lys Asn Val Leu Ser
    210                 215                 220

Leu Thr Asn Lys Gly Glu Val Phe Asn Glu Leu Val Gly Lys Gln Arg
225                 230                 235                 240

Ile Ser Gly Asn Leu Asp Ser Pro Glu Gly Gly Phe Asp Ala Ile Met
                245                 250                 255

Gln Val Ala Val Cys Gly Ser Leu Ile Gly Trp Arg Asn Val Thr Arg
            260                 265                 270

Leu Leu Val Phe Ser Thr Asp Ala Gly Phe His Phe Ala Gly Asp Gly
        275                 280                 285

Lys Leu Gly Gly Ile Val Leu Pro Asn Asp Gly Gln Cys His Leu Glu
    290                 295                 300

Asn Asn Met Tyr Thr Met Ser His Tyr Tyr Asp Tyr Pro Ser Ile Ala
305                 310                 315                 320

His Leu Val Gln Lys Leu Ser Glu Asn Asn Ile Gln Thr Ile Phe Ala
                325                 330                 335

Val Thr Glu Glu Phe Gln Pro Val Tyr Lys Glu Leu Lys Asn Leu Ile
            340                 345                 350

Pro Lys Ser Ala Val Gly Thr Leu Ser Ala Asn Ser Ser Asn Val Ile
        355                 360                 365

Gln Leu Ile Ile Asp Ala Tyr Asn Ser Leu Ser Ser Glu Val Ile Leu

370                 375                 380

Glu Asn Gly Lys Leu Ser Glu Gly Val Thr Ile Ser Tyr Lys Ser Tyr
385                 390                 395                 400

Cys Lys Asn Gly Val Asn Gly Thr Gly Glu Asn Gly Arg Lys Cys Ser
                405                 410                 415

Asn Ile Ser Ile Gly Asp Glu Val Gln Phe Glu Ile Ser Ile Thr Ser
            420                 425                 430

Asn Lys Cys Pro Lys Lys Asp Ser Asp Ser Phe Lys Ile Arg Pro Leu
        435                 440                 445

Gly Phe Thr Glu Glu Val Glu Val Ile Leu Gln Tyr Ile Cys Glu Cys
    450                 455                 460

Glu Cys Gln Ser Glu Gly Ile Pro Glu Ser Pro Lys Cys His Glu Gly
465                 470                 475                 480

Asn Gly Thr Phe Glu Cys Gly Ala Cys Arg Cys Asn Glu Gly Arg Val
                485                 490                 495

Gly Arg His Cys Glu Cys Ser Thr Asp Glu Val Asn Ser Glu Asp Met
            500                 505                 510

Asp Ala Tyr Cys Arg Lys Glu Asn Ser Ser Glu Ile Cys Ser Asn Asn
        515                 520                 525

Gly Glu Cys Val Cys Gly Gln Cys Val Cys Arg Lys Arg Asp Asn Thr
    530                 535                 540

Asn Glu Ile Tyr Ser Gly Lys Phe Cys Glu Cys Asp Asn Phe Asn Cys
545                 550                 555                 560

Asp Arg Ser Asn Gly Leu Ile Cys Gly Gly Asn Gly Val Cys Lys Cys
                565                 570                 575

Arg Val Cys Glu Cys Asn Pro Asn Tyr Thr Gly Ser Ala Cys Asp Cys
            580                 585                 590

Ser Leu Asp Thr Ser Thr Cys Glu Ala Ser Asn Gly Gln Ile Cys Asn
        595                 600                 605

Gly Arg Gly Ile Cys Glu Cys Gly Val Cys Lys Cys Thr Asp Pro Lys
    610                 615                 620

Phe Gln Gly Gln Thr Cys Glu Met Cys Gln Thr Cys Leu Gly Val Cys
625                 630                 635                 640

Ala Glu His Lys Glu Cys Val Gln Cys Arg Ala Phe Asn Lys Gly Glu

645                         650                         655

Lys Lys Asp Thr Cys Thr Gln Glu Cys Ser Tyr Phe Asn Ile Thr Lys
            660                         665                         670

Val Glu Ser Arg Asp Lys Leu Pro Gln Pro Val Gln Pro Asp Pro Val
        675                         680                         685

Ser His Cys Lys Glu Lys Asp Val Asp Asp Cys Trp Phe Tyr Phe Thr
    690                         695                         700

Tyr Ser Val Asn Gly Asn Asn Glu Val Met Val His Val Val Glu Asn
705                         710                         715                         720

Pro Glu Cys Pro Thr Gly Pro Asp Ile Ile Pro Ile Val Ala Gly Val
                725                         730                         735

Val Ala Gly Ile Val Leu Ile Gly Leu Ala Leu Leu Leu Ile Trp Lys
            740                         745                         750

Leu Leu Met Ile Ile His Asp Arg Arg Glu Phe Ala Lys Phe Glu Lys
        755                         760                         765

Glu Lys Met Asn Ala Lys Trp Asp Thr Pro Gly Val Gln Trp Cys Asp
    770                         775                         780

Ile Ser Ser Leu Gln Pro Leu Thr Ser Arg Phe Gln Gln Phe Ser Cys
785                         790                         795                         800

Leu Ser Leu Pro Ser Thr Trp Asp Tyr Arg Val Lys Ile Leu Phe Ile
                805                         810                         815

Arg Val Pro

<210>   13
<211>   801
<212>   PRT
<213>   Homo sapiens

<400>   13

Met Asn Leu Gln Pro Ile Phe Trp Ile Gly Leu Ile Ser Ser Val Cys
1               5                   10                  15

Cys Val Phe Ala Gln Thr Asp Glu Asn Arg Cys Leu Lys Ala Asn Ala
            20                  25                  30

Lys Ser Cys Gly Glu Cys Ile Gln Ala Gly Pro Asn Cys Gly Trp Cys
        35                  40                  45

Thr Asn Ser Thr Phe Leu Gln Glu Gly Met Pro Thr Ser Ala Arg Cys
    50                  55                  60

Asp Asp Leu Glu Ala Leu Lys Lys Lys Gly Cys Pro Pro Asp Asp Ile
65                  70                  75                  80

Glu Asn Pro Arg Gly Ser Lys Asp Ile Lys Lys Asn Lys Asn Val Thr
                85                  90                  95

Asn Arg Ser Lys Gly Thr Ala Glu Lys Leu Lys Pro Glu Asp Ile Thr
            100                 105                 110

Gln Ile Gln Pro Gln Gln Leu Val Leu Arg Leu Arg Ser Gly Glu Pro
        115                 120                 125

Gln Thr Phe Thr Leu Lys Phe Lys Arg Ala Glu Asp Tyr Pro Ile Asp
    130                 135                 140

Leu Tyr Tyr Leu Met Asp Leu Ser Tyr Ser Met Lys Asp Asp Leu Glu
145                 150                 155                 160

Asn Val Lys Ser Leu Gly Thr Asp Leu Met Asn Glu Met Arg Arg Ile
                165                 170                 175

Thr Ser Asp Phe Arg Ile Gly Phe Gly Ser Phe Val Glu Lys Thr Val
            180                 185                 190

Met Pro Tyr Ile Ser Thr Thr Pro Ala Lys Leu Arg Asn Pro Cys Thr
        195                 200                 205

Ser Glu Gln Asn Cys Thr Ser Pro Phe Ser Tyr Lys Asn Val Leu Ser
    210                 215                 220

Leu Thr Asn Lys Gly Glu Val Phe Asn Glu Leu Val Gly Lys Gln Arg
225                 230                 235                 240

Ile Ser Gly Asn Leu Asp Ser Pro Glu Gly Gly Phe Asp Ala Ile Met
                245                 250                 255

Gln Val Ala Val Cys Gly Ser Leu Ile Gly Trp Arg Asn Val Thr Arg
            260                 265                 270

Leu Leu Val Phe Ser Thr Asp Ala Gly Phe His Phe Ala Gly Asp Gly
        275                 280                 285

Lys Leu Gly Gly Ile Val Leu Pro Asn Asp Gly Gln Cys His Leu Glu
    290                 295                 300

Asn Asn Met Tyr Thr Met Ser His Tyr Tyr Asp Tyr Pro Ser Ile Ala
305                 310                 315                 320

His Leu Val Gln Lys Leu Ser Glu Asn Asn Ile Gln Thr Ile Phe Ala
                325                 330                 335

Val Thr Glu Glu Phe Gln Pro Val Tyr Lys Glu Leu Lys Asn Leu Ile
            340                 345                 350

Pro Lys Ser Ala Val Gly Thr Leu Ser Ala Asn Ser Ser Asn Val Ile
        355                 360                 365

Gln Leu Ile Ile Asp Ala Tyr Asn Ser Leu Ser Ser Glu Val Ile Leu
    370                 375                 380

Glu Asn Gly Lys Leu Ser Glu Gly Val Thr Ile Ser Tyr Lys Ser Tyr
385                 390                 395                 400

Cys Lys Asn Gly Val Asn Gly Thr Gly Glu Asn Gly Arg Lys Cys Ser
                405                 410                 415

Asn Ile Ser Ile Gly Asp Glu Val Gln Phe Glu Ile Ser Ile Thr Ser
            420                 425                 430

Asn Lys Cys Pro Lys Lys Asp Ser Asp Ser Phe Lys Ile Arg Pro Leu
        435                 440                 445

Gly Phe Thr Glu Glu Val Glu Val Ile Leu Gln Tyr Ile Cys Glu Cys
    450                 455                 460

Glu Cys Gln Ser Glu Gly Ile Pro Glu Ser Pro Lys Cys His Glu Gly
465                 470                 475                 480

Asn Gly Thr Phe Glu Cys Gly Ala Cys Arg Cys Asn Glu Gly Arg Val
                485                 490                 495

Gly Arg His Cys Glu Cys Ser Thr Asp Glu Val Asn Ser Glu Asp Met
            500                 505                 510

Asp Ala Tyr Cys Arg Lys Glu Asn Ser Ser Glu Ile Cys Ser Asn Asn
        515                 520                 525

Gly Glu Cys Val Cys Gly Gln Cys Val Cys Arg Lys Arg Asp Asn Thr
    530                 535                 540

Asn Glu Ile Tyr Ser Gly Lys Phe Cys Glu Cys Asp Asn Phe Asn Cys
545                 550                 555                 560

Asp Arg Ser Asn Gly Leu Ile Cys Gly Gly Asn Gly Val Cys Lys Cys
                565                 570                 575

Arg Val Cys Glu Cys Asn Pro Asn Tyr Thr Gly Ser Ala Cys Asp Cys
            580                 585                 590

Ser Leu Asp Thr Ser Thr Cys Glu Ala Ser Asn Gly Gln Ile Cys Asn
        595                 600                 605

Gly Arg Gly Ile Cys Glu Cys Gly Val Cys Lys Cys Thr Asp Pro Lys
    610                 615                 620

Phe Gln Gly Gln Thr Cys Glu Met Cys Gln Thr Cys Leu Gly Val Cys
625                 630                 635                 640

Ala Glu His Lys Glu Cys Val Gln Cys Arg Ala Phe Asn Lys Gly Glu
                645                 650                 655

Lys Lys Asp Thr Cys Thr Gln Glu Cys Ser Tyr Phe Asn Ile Thr Lys
            660                 665                 670

Val Glu Ser Arg Asp Lys Leu Pro Gln Pro Val Gln Pro Asp Pro Val
        675                 680                 685

Ser His Cys Lys Glu Lys Asp Val Asp Asp Cys Trp Phe Tyr Phe Thr
    690                 695                 700

Tyr Ser Val Asn Gly Asn Asn Glu Val Met Val His Val Val Glu Asn
705                 710                 715                 720

Pro Glu Cys Pro Thr Gly Pro Asp Ile Ile Pro Ile Val Ala Gly Val
                725                 730                 735

Val Ala Gly Ile Val Leu Ile Gly Leu Ala Leu Leu Leu Ile Trp Lys
            740                 745                 750

Leu Leu Met Ile Ile His Asp Arg Arg Glu Phe Ala Lys Phe Glu Lys
        755                 760                 765

Glu Lys Met Asn Ala Lys Trp Asp Thr Gln Glu Asn Pro Ile Tyr Lys
    770                 775                 780

Ser Pro Ile Asn Asn Phe Lys Asn Pro Asn Tyr Gly Arg Lys Ala Gly
785                 790                 795                 800

Leu

<210>  14
<211>  1066
<212>  PRT
<213>  Homo sapiens

<400>  14

Met Gly Pro Gly Pro Ser Arg Ala Pro Arg Ala Pro Arg Leu Met Leu
1               5                   10                  15

Cys Ala Leu Ala Leu Met Val Ala Ala Gly Gly Cys Val Val Ser Ala
            20                  25                  30

Phe Asn Leu Asp Thr Arg Phe Leu Val Val Lys Glu Ala Gly Asn Pro
        35                  40                  45

Gly Ser Leu Phe Gly Tyr Ser Val Ala Leu His Arg Gln Thr Glu Arg
    50                  55                  60

Gln Gln Arg Tyr Leu Leu Leu Ala Gly Ala Pro Arg Glu Leu Ala Val
65                  70                  75                  80

Pro Asp Gly Tyr Thr Asn Arg Thr Gly Ala Val Tyr Leu Cys Pro Leu
                85                  90                  95

Thr Ala His Lys Asp Asp Cys Glu Arg Met Asn Ile Thr Val Lys Asn
            100                 105                 110

Asp Pro Gly His His Ile Ile Glu Asp Met Trp Leu Gly Val Thr Val
        115                 120                 125

Ala Ser Gln Gly Pro Ala Gly Arg Val Leu Val Cys Ala His Arg Tyr
    130                 135                 140

Thr Gln Val Leu Trp Ser Gly Ser Glu Asp Gln Arg Arg Met Val Gly
145                 150                 155                 160

Lys Cys Tyr Val Arg Gly Asn Asp Leu Glu Leu Asp Ser Ser Asp Asp
                165                 170                 175

Trp Gln Thr Tyr His Asn Glu Met Cys Asn Ser Asn Thr Asp Tyr Leu
            180                 185                 190

Glu Thr Gly Met Cys Gln Leu Gly Thr Ser Gly Gly Phe Thr Gln Asn
        195                 200                 205

Thr Val Tyr Phe Gly Ala Pro Gly Ala Tyr Asn Trp Lys Gly Asn Ser
    210                 215                 220

Tyr Met Ile Gln Arg Lys Glu Trp Asp Leu Ser Glu Tyr Ser Tyr Lys
225                 230                 235                 240

Asp Pro Glu Asp Gln Gly Asn Leu Tyr Ile Gly Tyr Thr Met Gln Val
                245                 250                 255

Gly Ser Phe Ile Leu His Pro Lys Asn Ile Thr Ile Val Thr Gly Ala
            260                 265                 270

Pro Arg His Arg His Met Gly Ala Val Phe Leu Leu Ser Gln Glu Ala
        275                 280                 285

Gly Gly Asp Leu Arg Arg Arg Gln Val Leu Glu Gly Ser Gln Val Gly
    290                 295                 300

Ala Tyr Phe Gly Ser Ala Ile Ala Leu Ala Asp Leu Asn Asn Asp Gly
305                 310                 315                 320

Trp Gln Asp Leu Leu Val Gly Ala Pro Tyr Tyr Phe Glu Arg Lys Glu
                325                 330                 335

Glu Val Gly Gly Ala Ile Tyr Val Phe Met Asn Gln Ala Gly Thr Ser
            340                 345                 350

Phe Pro Ala His Pro Ser Leu Leu Leu His Gly Pro Ser Gly Ser Ala
        355                 360                 365

Phe Gly Leu Ser Val Ala Ser Ile Gly Asp Ile Asn Gln Asp Gly Phe
    370                 375                 380

Gln Asp Ile Ala Val Gly Ala Pro Phe Glu Gly Leu Gly Lys Val Tyr
385                 390                 395                 400

Ile Tyr His Ser Ser Ser Lys Gly Leu Leu Arg Gln Pro Gln Gln Val
                405                 410                 415

Ile His Gly Glu Lys Leu Gly Leu Pro Gly Leu Ala Thr Phe Gly Tyr
            420                 425                 430

Ser Leu Ser Gly Gln Met Asp Val Asp Glu Asn Phe Tyr Pro Asp Leu
        435                 440                 445

Leu Val Gly Ser Leu Ser Asp His Ile Val Leu Leu Arg Ala Arg Pro
    450                 455                 460

Val Ile Asn Ile Val His Lys Thr Leu Val Pro Arg Pro Ala Val Leu
465                 470                 475                 480

Asp Pro Ala Leu Cys Thr Ala Thr Ser Cys Val Gln Val Glu Leu Cys
                485                 490                 495

Phe Ala Tyr Asn Gln Ser Ala Gly Asn Pro Asn Tyr Arg Arg Asn Ile
            500                 505                 510

Thr Leu Ala Tyr Thr Leu Glu Ala Asp Arg Asp Arg Arg Pro Pro Arg
        515                 520                 525

Leu Arg Phe Ala Gly Ser Glu Ser Ala Val Phe His Gly Phe Phe Ser
    530                 535                 540

Met Pro Glu Met Arg Cys Gln Lys Leu Glu Leu Leu Leu Met Asp Asn
545                 550                 555                 560

Leu Arg Asp Lys Leu Arg Pro Ile Ile Ile Ser Met Asn Tyr Ser Leu
                565                 570                 575

Pro Leu Arg Met Pro Asp Arg Pro Arg Leu Gly Leu Arg Ser Leu Asp
            580                 585                 590

Ala Tyr Pro Ile Leu Asn Gln Ala Gln Ala Leu Glu Asn His Thr Glu
        595                 600                 605

Val Gln Phe Gln Lys Glu Cys Gly Pro Asp Asn Lys Cys Glu Ser Asn
    610                 615                 620

Leu Gln Met Arg Ala Ala Phe Val Ser Glu Gln Gln Gln Lys Leu Ser
625                 630                 635                 640

Arg Leu Gln Tyr Ser Arg Asp Val Arg Lys Leu Leu Leu Ser Ile Asn
                645                 650                 655

Val Thr Asn Thr Arg Thr Ser Glu Arg Ser Gly Glu Asp Ala His Glu
            660                 665                 670

Ala Leu Leu Thr Leu Val Val Pro Pro Ala Leu Leu Leu Ser Ser Val
        675                 680                 685

Arg Pro Pro Gly Ala Cys Gln Ala Asn Glu Thr Ile Phe Cys Glu Leu
    690                 695                 700

Gly Asn Pro Phe Lys Arg Asn Gln Arg Met Glu Leu Leu Ile Ala Phe
705                 710                 715                 720

Glu Val Ile Gly Val Thr Leu His Thr Arg Asp Leu Gln Val Gln Leu
                725                 730                 735

Gln Leu Ser Thr Ser Ser His Gln Asp Asn Leu Trp Pro Met Ile Leu
            740                 745                 750

Thr Leu Leu Val Asp Tyr Thr Leu Gln Thr Ser Leu Ser Met Val Asn
        755                 760                 765

His Arg Leu Gln Ser Phe Phe Gly Gly Thr Val Met Gly Glu Ser Gly
    770                 775                 780

Met Lys Thr Val Glu Asp Val Gly Ser Pro Leu Lys Tyr Glu Phe Gln
785                 790                 795                 800

Val Gly Pro Met Gly Glu Gly Leu Val Gly Leu Gly Thr Leu Val Leu
                805                 810                 815

Gly Leu Glu Trp Pro Tyr Glu Val Ser Asn Gly Lys Trp Leu Leu Tyr
            820                 825                 830

Pro Thr Glu Ile Thr Val His Gly Asn Gly Ser Trp Pro Cys Arg Pro
        835                 840                 845

Pro Gly Asp Leu Ile Asn Pro Leu Asn Leu Thr Leu Ser Asp Pro Gly
    850                 855                 860

Asp Arg Pro Ser Ser Pro Gln Arg Arg Arg Arg Gln Leu Asp Pro Gly
865                 870                 875                 880

Gly Gly Gln Gly Pro Pro Pro Val Thr Leu Ala Ala Ala Lys Lys Ala
                885                 890                 895

Lys Ser Glu Thr Val Leu Thr Cys Ala Thr Gly Arg Ala His Cys Val
            900                 905                 910

Trp Leu Glu Cys Pro Ile Pro Asp Ala Pro Val Val Thr Asn Val Thr
        915                 920                 925

Val Lys Ala Arg Val Trp Asn Ser Thr Phe Ile Glu Asp Tyr Arg Asp
    930                 935                 940

Phe Asp Arg Val Arg Val Asn Gly Trp Ala Thr Leu Phe Leu Arg Thr
945                 950                 955                 960

Ser Ile Pro Thr Ile Asn Met Glu Asn Lys Thr Thr Trp Phe Ser Val
                965                 970                 975

Asp Ile Asp Ser Glu Leu Val Glu Glu Leu Pro Ala Glu Ile Glu Leu
            980                 985                 990

Trp Leu Val Leu Val Ala Val Gly Ala Gly Leu Leu Leu Leu Gly Leu
        995                 1000                1005

Ile Ile Leu Leu Leu Trp Lys Cys Asp Phe Phe Lys Arg Thr Arg
    1010                1015                1020

Tyr Tyr Gln Ile Met Pro Lys Tyr His Ala Val Arg Ile Arg Glu
    1025                1030                1035

Glu Glu Arg Tyr Pro Pro Pro Gly Ser Thr Leu Pro Thr Lys Lys
    1040                1045                1050

His Trp Val Thr Ser Trp Gln Thr Arg Asp Gln Tyr Tyr
    1055                1060                1065